# EUROPEAN PATENT APPLICATION

(11) **EP 4 063 382 A1**
(43) Date of publication of application: **28.09.2022**
(21) Application number: 20941459.8
(22) Date of filing: 19.06.2020
(51) Int. Cl.: C07K 14/35, C12N 9/00, C12N 15/31, C12Q 1/6869

(54) **PIF1-LIKE HELICASE AND APPLICATION THEREOF**

(71) Applicant: Qitan Technology Ltd., Beijing, Beijing 100192 (CN)
(72) Inventor: ZHANG, Zhougang, Beijing 100192 (CN); WANG, Muyang, Beijing 100192 (CN); GUO, Qianqian, Beijing 100192 (CN); CHEN, Chengyao, Beijing 100192 (CN)
(74) Representative: Boult Wade Tennant LLP
(86) International application number: PCT/CN2020/097126
(87) International publication number: WO 2021/253410

(57) **Abstract**

A modified Pifl-like helicase, a construct containing a Pifl-like helicase, and an application thereof in characterizing a target polynucleotide or controlling the movement of a target polynucleotide passing through pores. The present invention also relates to a method for characterizing a target polynucleotide or controlling the movement of a target polynucleotide passing through pores. The Pifl-like helicase can effectively control the movement of a polynucleotide passing through pores and increase the accuracy of a chain sequencing process.

## Description

### TECHNICAL FIELD

The present application relates to the technical fields of gene sequencing, molecular detection and clinical detection, particularly relates to a modified Pif1-like helicase, a construct comprising the Pifl-like helicase, and its use in characterising a target polynucleotide or controlling the movement of a target polynucleotide through a pore.

### BACKGROUND

Nanopore sequencing technology refers to a gene sequencing technology that uses a single nucleic acid molecule as a measurement unit and uses nanopores to read its sequence information in real time and continuously.

The nanopore sequencing technology uses nanopores that can provide channels for ion current. Electrophoresis drives polynucleotides through the nanopore. Since a polynucleotide passes through the nanopore, the current passing through the nanopore may be reduced. Each nucleotide or series of nucleotides passing through the nanopore produce a characteristic current, and the current level corresponds to the polynucleotide sequence. In "strand sequencing" (such as using a helicase to control the movement of a polynucleotide through a pore), a single polynucleotide chain passes through the pore and enables identification of nucleotides.

The sequencing technology has many advantages, such as the library may be easily constructed without amplification; the technology has a fast reading speed, and may reach a reading speed of tens of thousands of bases per hour for a single molecule; the technology has a long read length, usually thousands of bases; and it is possible to make a direct measurement of RNA and DNA methylation. These are beyond the reach of the existing second-generation sequencing technology.

However, nanopore sequencing technology also has difficult problems that need to be solved. For example, the translocation of polynucleotides through the nanopore is so fast that the current level of a single nucleotide is too short to be distinguished. Especially when the nucleotide sequence has a very long length, such as 500 or more nucleotides, the molecular motor which is controlling the movement of the polynucleotide may disengage from the polynucleotide. This allows the polynucleotide to be pulled through the pore rapidly and in an uncontrolled manner in the direction of the applied field.

There are existing technologies that can solve this problem. For example, patent application WO2013057495A3 discloses a new method of characterising a target polynucleotide, wherein the method comprises controlling the movement of the target polynucleotide through a pore by Hel308 helicase or molecular motors. Patent application US20150065354A1 discloses a method of characterising a target polynucleotide by using XPD helicase, wherein the method comprises controlling the movement of the target polynucleotide through pores by XPD helicase. Patent application CN107109380A discloses a modified enzyme, which is a modified Dda helicase that can control the movement of a target polynucleotide through a pore. All of the above methods may control the movement of a target polynucleotide through a pore to a certain extent. However, there is still a need to develop a new helicase that controls the movement of a target polynucleotide through a pore.

### SUMMARY

The present application provides a new modified Pifl-like helicase to solve the problem of too fast translocation of polynucleotides through a nanopore. The modified Pifl-like helicase may maintain the binding to the polynucleotide for a longer time and control the movement of the polynucleotide through the pore. The Pifl-like helicase of the present application is a useful tool for controlling the movement of polynucleotides in the strand sequencing, causing the polynucleotides to move in a controlled and stepwise manner along or against the electric field caused by the applied voltage, thereby controlling the speed of the polynucleotides passing through the nanopore, and obtaining a recognizable current level. Especially when the polynucleotide chain has an increased length, such as 500 or more nucleotides, and molecular motors are required with improved processivity, the Pifl-like helicase of the present application will still not disengage from the polynucleotide, that is, it is particularly effective in controlling the movement of polynucleotides of 500, 1000, 5000, 10000, 20000, 50000, 100000 or more nucleotides. The Pifl-like helicase of the present application has the advantages of controlling the smooth movement of polynucleotides from a pore and reducing slippage or irregular movement, thereby promoting more accurate reading of nucleotides and longer read lengths.

In the first aspect of the present application, provided is a Pifl-like helicase comprising at least one cysteine residue and/or at least one non-natural amino acid introduced into a tower domain, a pin domain and/or a 1A (RecA-like motor) domain of the Pifl-like helicase, wherein the Pifl-like helicase retains its ability to control the movement of a polynucleotide.

Preferably, in the Pifl-like helicase the at least one cysteine residue and/or at least one non-natural amino acid have been introduced into any one selected from the group consisting of the following:
(A) the tower domain;
(B) the pin domain;
(C) the 1A domain;
(D) the tower domain and the pin domain;
(E) the tower domain and the 1A domain;
(F) the 1A domain and the pin domain;
(G) the tower domain, the pin domain and the 1A domain.

Preferably, into the tower domain, the pin domain and/or the 1A domain, 1, 2, 3, 4, 5, 6, 7, 8, 9, 10, 11 or more cysteine residues may be introduced or 1, 2, 3, 4, 5, 6, 7, 8, 9, 10, 11 or more non-natural amino acids may be introduced, or 1, 2, 3, 4, 5, 6, 7, 8, 9, 10, 11 or more cysteine residues and non-natural amino acids may be introduced.

Preferably, the introduction of cysteine residues and/or at least one non-natural amino acids make the binding of the Pifl-like helicase to the polynucleotide more stable and enhances its ability to control the movement.

Preferably, the Pifl-like helicase is selected from Pba-PM2, Aph-Acj61, Aph-PX29, Avi-Aehl, Sph-CBH8, Eph-Pei26, Aph-AM101, PphPspYZU05, Eph-EcS1, Eph- Cronus2 or Mph-MP1. Some wild-type Pifl-like helicases are shown in Table 1.

**Table 1. Wild-type Pifl-like helicases**

| Wild-type Pifl-like helicase | | UniProt | Length |
|---|---|---|---|
| Pba-PM2 (SEQ ID NO:1) | Pectobacterium bacteriophage PM2 | A0A0A0Q3A4 | 444 |
| Aph-Acj61 (SEQ ID NO:2) | Acinetobacter phage Acj61 | E5E403 | 441 |
| Aph-PX29 (SEQ ID NO:3) | Aeromonas phage PX29 | E5DQH4 | 452 |
| Avi-Aehl (SEQ ID NO:4) | Aeromonas virus Aeh1 | Q76YI7 | 453 |
| Sph-CBH8 (SEQ ID NO:5) | Serratia phage CBH8 | A0A1Z1LY03 | 441 |
| Eph-Pei26 (SEQ ID NO:6) | Edwardsiella phage PEi26 | A0A0B6VP37 | 446 |
| Aph-AM101 (SEQ ID NO:7) | Acinetobacter phage AM101 | A0A4Y1NMU3 | 452 |
| PphPspYZU05 (SEQ ID NO:8) | Pseudomonas phage PspYZU05 | A0A2U7NRX4 | 454 |
| Eph-EcS1 (SEQ ID NO:9) | Escherichia phage EcS 1 | A0A2Z5ZC85 | 441 |
| Eph-Cronus2 (SEQ ID NO:10) | Erwinia phage Cronus | A0A2S1GM41 | 443 |
| Mph-MP1 (SEQ ID NO:11) | Morganella phage vB_MmoM_MP1 | A0A192Y9K3 | 441 |

The amino acid residue positions of the tower domain, the pin domain, the hook domain, the 1A domain and the 2A domain of the Pifl-like helicase are shown in Table 2.

**Table 2 Residues making up each domain in each Pifl-like helicase as identified.**

| Pifl-like helicase | SEQ ID NO | 1A domain | 2 A domain |
|---|---|---|---|
| Pba-PM2 | 1 | M1-L88, M106-V181 | E264-P278, N296-A394 |
| Aph-Acj61 | 2 | M1-L88, I106-M180 | E265-P279, N297-A392 |
| Aph-PX29 | 3 | M1-L88, K110-V182 | T266-P280, N298-S403 |
| Avi-Aehl | 4 | M1-L88, K110-V182 | T266-P280, N298-S404 |
| Sph-CBH8 | 5 | M1-L84, M103-K177 | E260-P274, N292-A391 |
| Eph-Pei26 | 6 | M1-L90, M108-M183 | E266-P280, N298-A396 |
| Aph-AM101 | 7 | M1-L99, D117-M191 | T276-P290, N308-P402 |
| PphPspYZU05 | 8 | M1-L95, I113-K187 | D274-P288, N306-A404 |
| Eph-EcS1 | 9 | M1-L85, M103-K177 | E260-P274, N292-A391 |
| Eph-Cronus2 | 10 | M1-L87, I105-K180 | E265-P279, H297-A393 |
| Mph-MP1 | 11 | M1-L96, P114-K184 | E264-P278, N296-P389 |

| Pifl-like helicase | SEQ ID NO | Pin domain | Tower domain | Hook domain |
|---|---|---|---|---|
| Pba-PM2 | 1 | K89-E105 | E264-P278, N296-A394 | E277-F295 |
| Aph-Acj61 | 2 | K89-D105 | E265-P279, N297-A392 | E278-F296 |
| Aph-PX29 | 3 | K89-A109 | T266-P280, N298-S403 | E279-L297 |
| Avi-Aehl | 4 | K89-A109 | T266-P280, N298-S404 | E279-L297 |
| Sph-CBH8 | 5 | K86-E102 | E260-P274, N292-A391 | E273-F291 |
| Eph-Pei26 | 6 | K91-E107 | E266-P280, N298-A396 | E279-F297 |
| Aph-AM101 | 7 | K100-D116 | T276-P290, N308-P402 | E289-F307 |
| PphPspYZU05 | 8 | K95-E112 | D274-P288, N306-A404 | G287-Y305 |
| Eph-EcS1 | 9 | K86-E102 | E260-P274, N292-A391 | E273-F291 |
| Eph-Cronus2 | 10 | K88-E104 | E265-P279, H297-A393 | E278-F296 |
| Mph-MP1 | 11 | K97-A113 | E264-P278, N296-P389 | E277-F295 |

In a specific embodiment of the present application, the Pif1-like helicase is selected from Mph-MP1, Sph-CBH8, Eph-Pei26 or PphPspYZU05.

Preferably, the helicase comprises a variant of SEQ ID NO: 11 in which at least one cysteine residue and/or at least one non-natural amino acid have been introduced into the tower domain (residues E264-P278 and N296-P389), and/or the pin domain (residues K97-A113), and/or the 1A domain (residues M1-L96 and P114-K184).

Preferably, the helicase comprises a variant of SEQ ID NO: 1 in which at least one cysteine residue and/or at least one non-natural amino acid have been introduced into the tower domain (residues E264-P278 and N296-A394), and/or the pin domain (residues K89-E105), and/or the 1A domain (residues M1-L88 and M106-V181).

Preferably, the helicase comprises a variant of SEQ ID NO: 2 in which at least one cysteine residue and/or at least one non-natural amino acid have been introduced into the tower domain (residues E265-P279 and N297-A392), and/or the pin domain (residue K89-D105), and/or the 1A domain (residue M1-L88 and I106-M180).

Preferably, the helicase comprises a variant of SEQ ID NO: 3 in which at least one cysteine residue and/or at least one non-natural amino acid have been introduced into the tower domain (residues T266-P280 and N298-S403), and/or the pin domain (residues K89-A109), and/or the 1A domain (residues M1-L88 and K110-V182).

Preferably, the helicase comprises a variant of SEQ ID NO: 4 in which at least one cysteine residue and/or at least one non-natural amino acid have been introduced into the tower domain (residues T266-P280 and N298-S404), and/or the pin domain (residues K89-A109), and/or the 1A domain (residues M1-L88 and K110-V182).

Preferably, the helicase comprises a variant of SEQ ID NO: 5 in which at least one cysteine residue and/or at least one non-natural amino acid have been introduced into the tower domain (residues E260-P274 and N292-A391), and/or the pin domain (residues K86-E102), and/or the 1A domain (residues M1-L84 and M103-K177).

Preferably, the helicase comprises a variant of SEQ ID NO: 6 in which at least one cysteine residue and/or at least one non-natural amino acid have been introduced into the tower domain (residues E266-P280 and N298-A396), and/or the pin domain (residues K91-E107), and/or the 1A domain (residues M1-L90 and M108-M183).

Preferably, the helicase comprises a variant of SEQ ID NO: 7 in which at least one cysteine residue and/or at least one non-natural amino acid have been introduced into the tower domain (residues T276-P290 and N308-P402), and/or the pin domain (residues K100-D116), and/or the 1A domain (residues M1-L99 and D117-M191).

Preferably, the helicase comprises a variant of SEQ ID NO: 8 in which at least one cysteine residue and/or at least one non-natural amino acid have been introduced into the tower domain (residues D274-P288 and N306-A404), and/or the pin domain (residues K95-E112), and/or the 1A domain (residues M1-L95 and I113-K187).

Preferably, the helicase comprises a variant of SEQ ID NO: 9 in which at least one cysteine residue and/or at least one non-natural amino acid have been introduced into the tower domain (residues E260-P274 and N292-A391), and/or the pin domain (residues K86-E102), and/or the 1A domain (residues M1-L85 and M103-K177).

Preferably, the helicase comprises a variant of SEQ ID NO: 10 in which at least one cysteine residue and/or at least one non-natural amino acid have been introduced into the tower domain (residues E265-P279 and H297-A393), and/or the pin domain (residues K88-E104), and/or the 1A domain (residues M1-L87 and I105-K180).

Preferably, the helicase comprises a variant of SEQ ID NO: 11, which comprises (i) E105C and/or A362C; (ii) E104C and/or K360C; (iii) E104C and/or A362C; (iv) E104C and/or Q363C; (v) E104C and/or K366C; (vi) E105C and/or M356C; (vii) E105C and/or K360C; (viii) E104C and/or M356C; (ix) E105C and/or Q363C; (x) E105C and/or K366C; (xi) F108C and/or M356C; (xii) F108C and/or K360C; (xiii) F108C and/or A362C; (xiv) F108C and/or Q363C; (xv) F108C and/or K366C; (xvi) K134C and/or M356C; (xvii) K134C and/or K360C; (xviii) K134C and/or A362C; (xix) K134C and/or Q363C; (xx) K134C and/or K366C; (xxi) any of (i) to (xx) and G359C; (xxii) any of (i) to (xx) and Q111C; (xxiii) any of (i) to (xx) and I138C; (xxiv) any of (i) to (xx) and Q111C and I138C; (xxv) E105C and/or F377C; (xxvi) Y103L, E105Y, N352N, A362C and Y365N; (xxvii) E105Y and A362C; (xxviii) A362C; (xxix) Y103L, E105C, N352N, A362Y and Y365N; (xxx) Y103L, E105C and A362Y; (xxxi) E105C and/or A362C, and I280A; (xxxii) E105C and/or L358C; (xxxiii) E104C and/or G359C; (xxxiv) E104C and/or A362C ; (Xxxv) K106C and/or W378C; (xxxvi) T102C and/or N382C; (xxxvii) T102C and/or W378C; (xxxviii) E104C and/or Y355C; (xxxix) E104C and/or N382C; (xl) E104C and/or K381C; (xli) E104C and/or K379C; (xlii) E104C and/or D376C; (xliii) E104C and/or W378C; (xliv) E104C and/or W374C; (xlv) E105C and/or Y355C; (xlvi) E105C and/or N382C; (xlvii) E105C and/or K381C; (xlviii) E105C and/or K379C; (xlix) E105C and/or D376C; (1) E105C and/or W378C; (li) E105C and/or W374C; (lii) E105C and A362Y; (liii) E105C, G359C and A362C; or (liv)I2C, E105C and A362C.

Preferably, the helicase comprises a variant of any one of SEQ ID NOs: 1 to 10, which comprises a cysteine residue at the positions which correspond to those in SEQ ID NO: 11 as defined in any of (i) to (liv).

Preferably, the amino acid sequence of the Pifl-like helicase is one of the amino acid sequences shown in SEQ ID NOs:1 to 11 or has at least 30%, at least 40%, at least 50%, 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology with one of the amino acid sequences shown in SEQ ID NOs: 1 to 11, and has the ability to control the movement of a polynucleotide.

In a specific embodiment of the present application, a cysteine residue has been introduced at the amino acid position(s) corresponding to position(s) 10 and/or 362 of SEQ ID NO: 11, for example, position(s) 97 and/or 363 of SEQ ID NO: 2, position(s) 96 and/or 371 of SEQ ID NO: 3, position(s) 94 and/or 361 of SEQ ID NO: 5, position(s) 99 and/or 366 of SEQ ID NO: 6, and position(s) 104 and/or 375 of SEQ ID NO: 8, and the like.

In order to improve the stability of the Pifl-like helicase of the present application binding to a polynucleotide, and decrease the ability of the helicase to disengage from the polynucleotide, the introduced cysteines are connected to one another, the introduced non-natural amino acids are connected to one another, the introduced cysteine and the introduced non-natural amino acid are connected to one another, the introduced cysteine and a native amino acid of the helicase are connected to one another, or the introduced non-natural amino acid and a native amino acid of the helicase are connected to one another.

Any number and combination of two more of the introduced cysteines and/or non-natural amino acids may be connected to one another. For instance, 3, 4, 5, 6, 7, 8 or more cysteines and/or non-natural amino acids may be connected to one another. One or more cysteines may be connected to one or more cysteines. One or more cysteines may be connected to one or more non-natural amino acids, such as Faz. One or more non-natural amino acids, such as Faz, may be connected to one or more non-natural amino acids, such as Faz. One or more cysteines may be connected to one or more native amino acids of the helicase. One or more non-natural amino acids, such as Faz may be connected to one or more native amino acids of the helicase.

The connection may be in any manner, including direct connection or indirect connection. Preferably, the connection may be a transient contact or a permanent connection. More preferably, the connection may be a non-covalent connection or a covalent connection.

In a specific embodiment of the present application, the covalent connection may be implemented by using chemical crosslinkers, linear molecules or catalysts. The chemical crosslinkers include, but are not limited to, maleimide, active esters, succinimide, azides, alkynes (such as dibenzocyclooctynol (DIBO or DBCO), difluoro cycloalkynes and linear alkynes), and the like. The chemical crosslinkers may vary in length from one carbon (phosgene-type linkers) to many Angstroms. Examples of linear molecules, include but are not limited to, are polyethyleneglycols (PEGs), polypeptides, polysaccharides, deoxyribonucleic acid (DNA), peptide nucleic acid (PNA), threose nucleic acid (TNA), glycerol nucleic acid (GNA), saturated and unsaturated hydrocarbons, polyamides. The catalysts include, but are not limited to, any catalysts, such as TMAD, that can produce covalent bonds between cysteine residues, between non-natural amino acids, between cysteine residues and non-natural amino acids, between non-natural amino acids and native amino acids or between cysteine residues and native amino acids.

Preferably, the Pif1-like helicase is further modified by removal of one or more cysteine residues.

Preferably, the Pifl-like helicase further comprises a substitution of at least one or more native cysteine residues; more preferably the cysteine residues being substituted with alanine, serine or valine.

Preferably, the Pifl-like helicase comprises a variant of SEQ ID NO: 5, and the one or more substituted native cysteine residues are one or more of C109, C114, C136 or C414.

Preferably, the Pifl-like helicase comprises a variant of any one of SEQ ID NOs: 1, 2, 3, 4, 6, 7, 8, 9, 10, and 11, and the one or more substituted native cysteine residues correspond to one or more of C109, C114, C136 or C414 in SEQ ID NO:5.

Table 3 shows the amino acid positions in SEQ ID NOs: 1, 2, 3, 4, 6, 7, 8, 9, 10, and 11 corresponding to C109, C114, C136, and C414 in SEQ ID NO: 5.

**Table 3. Corresponding amino acid positions in SEQ ID NOs: 1-4, and 6-11**

| SEQ ID NO: | Amino acid positions | | | |
|---|---|---|---|---|
| 5 | C109 | C114 | C136 | C414 |
| 1 | C112 | C118 | C139 | C417 |
| 2 | C113 | C117 | C139 | C415 |
| 3 | - | - | C143 | - |
| 4 | - | - | C144 | - |
| 6 | C114 | C119 | C141 | C420 |
| 7 | C124 | C128 | C150 | C426 |
| 8 | C119 | C124 | C146 | |
| 9 | C109 | C114 | C136 | C414 |
| 10 | C111 | C116 | C138 | C416 |
| 11 | - | C125 | - | C412 |

Preferably, the helicase is further modified to reduce its surface negative charge.

Preferably, the Pifl-like helicase further comprises a substitution increasing the net positive charge. More preferably, the substitution increasing the net positive charge comprises a substitution or modification of a negatively charged amino acid, a polar or non-polar amino acid, or introduction a positively charged amino acid at positions near a surface negatively-charged amino acid, a polar or non-polar amino acid. More preferably, the substitution increasing the net positive charge comprises a substitution of negatively charged amino acids, uncharged amino acids, aromatic amino acids, polar or non-polar amino acids with positively charged amino acids. More preferably, the substitution increasing the net positive charge comprises a substitution of negatively charged amino acids, aromatic amino acids, polar or non-polar amino acids with uncharged amino acids. Suitable positively charged amino acids include, but are not limited to, histidine (H), lysine (K) and/or arginine (R). Uncharged amino acids have no net charge. Suitable uncharged amino acids include, but are not limited to, cysteine (C), serine (S), threonine (T), methionine (M), asparagine (N) or glutamine (Q). Non-polar amino acids have non-polar side chains. Non-polar amino acids include, but are not limited to, glycine (G), alanine (A), proline (P), isoleucine (I), leucine (L) or valine (V). Aromatic amino acids have aromatic side chains. Suitable aromatic amino acids include, but are not limited to, histidine (H), phenylalanine (F), tryptophan (W) or tyrosine (Y).

The positively charged amino acids, uncharged amino acids, polar, non-polar amino acids, or aromatic amino acids may be natural or non-natural amino acids, which may be artificially synthesized or modified natural amino acids.

Preferred substitutions include, but are not limited to, a substitution of glutamic acid (E) with arginine (R), a substitution of glutamic acid (E) with lysine (K), and a substitution of glutamic acid (E) with asparagine (N), a substitution of aspartic acid (D) with lysine (K), and a substitution of aspartic acid (D) with arginine (R).

Preferably, the Pifl-like helicase comprises a variant of SEQ ID NO: 11, and the one or more negatively charged amino acids are one or more of D5, E9, E24, E87, 165, S58, D209 or D216. Any number of these amino acids may be neutralised, such as 1, 2, 3, 4, 5, 6, 7 or 8 of them. Any combination may be neutralised.

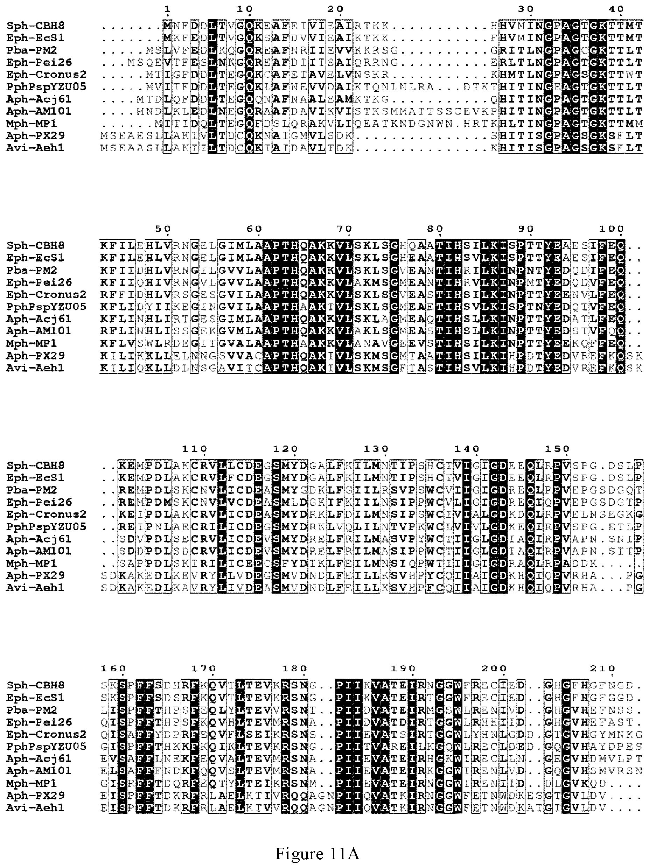

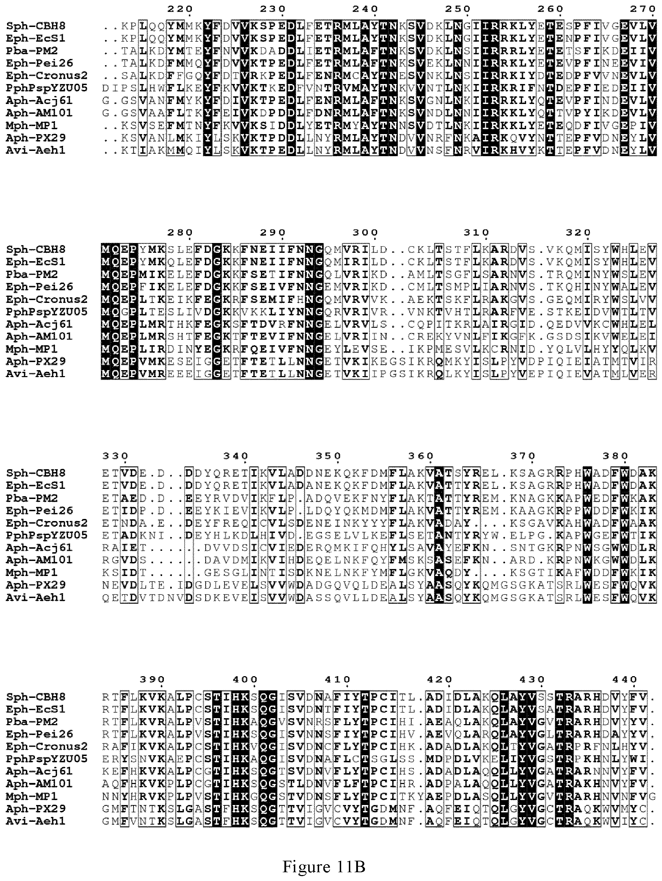

Preferably, the Pifl-like helicase comprises a variant of any one of SEQ ID NOs: 1 to 10, and the one or more negatively charged amino acids correspond to one or more of D5, E9, E24, E87, I65, S58, D209, or D216 in SEQ ID NO: 11. Amino acids in SEQ ID NOs: 1 to 10 which correspond to D5, E9, E24, E87, 165, S58, D209, or D216 in SEQ ID NO: 11 may be determined using the alignment shown in Figure 11.

Preferred substitution also comprises a variant of SEQ ID NO: 1, and the one or more negatively charged amino acids further comprise S171.

Preferred substitution also comprises a variant of SEQ ID NO: 9, and the one or more negatively charged amino acids further comprise S173.

Preferably, the non-natural amino acid is selected from 4-Azido-L-phenylalanine (Faz), 4-Acetyl-L-phenylalanine, 3-Acetyl-L-phenylalanine, 4-Acetoacetyl-L-phenylalanine, O-Allyl-L-tyrosine, 3-(Phenylselanyl)-L-alanine, O-2-Propyn-l-yl-L-tyrosine, 4-(Dihydroxyboryl)-L-phenylalanine, 4-[(Ethylsulfanyl)carbonyl]-L-phenylalanine, (2S)-2-amino-3-{4-[(propan-2-ylsulfanyl)carbonyl]phenyl}propanoic acid, (2*S*)-2-amino-3-{4-[(2-amino-3-sulfanylpropanoyl)amino]phenyl}propanoic acid, O-Methyl-L-tyrosine, 4-Amino-L-phenylalanine, 4-Cyano-L-phenylalanine, 3-Cyano-L-phenylalanine, 4-Fluoro-L-phenylalanine, 4-Iodo-L-phenylalanine, 4-Bromo-L-phenylalanine, O-(Trifluoromethyl)tyrosine, 4-Nitro-L-phenylalanine, 3-Hydroxy-L-tyrosine, 3-Amino-L-tyrosine, 3-Iodo-L-tyrosine, 4-Isopropyl-L-phenylalanine, 3-(2-Naphthyl)-L-alanine, 4-Phenyl-L-phenylalanine, (2*S*)-2-amino-3-(naphthalen-2-ylamino)propanoic acid, 6-(Methylsulfanyl)norleucine, 6-Oxo-L-lysine, D-tyrosine, (2R)-2-Hydroxy-3-(4-hydroxyphenyl)propanoic acid, (2R)-2-Ammoniooctanoate3-(2,2'-Bipyridin-5-yl)-D-alanine, 2-amino-3-(8-hydroxy-3-quinolyl)propanoic acid, 4-Benzoyl-L-phenylalanine, S-(2-Nitrobenzyl)cysteine, (2R)-2-amino-3-[(2-nitrobenzyl)sulfanyl]propanoic acid, (2*S*)-2-amino-3-[(2-nitrobenzyl)oxy]propanoic acid, O-(4,5-Dimethoxy-2-nitrobenzyl)-L-serine, (2*S*)-2-amino-6-({[(2-nitrobenzyl)oxy]carbonyl}amino)hexanoic acid, O-(2-Nitrobenzyl)-L-tyrosine, 2-Nitrophenylalanine, 4-[(E)-Phenyldiazenyl]-L-phenylalanine, 4-[3-(Trifluoromethyl)-3H-diaziren-3-yl]-D-phenylalanine, 2-amino-3-[[5-(dimethylamino)-l-naphthyl]sulfonylamino]propanoic acid, (2*S*)-2-amino-4-(7-hydroxy-2-oxo-2H-chromen-4-yl)butanoic acid, (2*S*)-3-[(6-acetylnaphthalen-2-yl)amino]-2-aminopropanoic acid, 4-(Carboxymethyl)phenylalanine, 3-Nitro-L-tyrosine, O-Sulfo-L-tyrosine, (2R)-6-Acetamido-2-ammoniohexanoate, 1-Methylhistidine, 2-Aminononanoic acid, 2-Aminodecanoic acid, L-Homocysteine, 5-Sulfanylnorvaline, 6-Sulfanyl-L-norleucine, 5-(Methyl sulfanyl)-L-norvaline, N⁶-{[(2R,3R)-3-Methyl-3,4-dihydro-2H-pyrrol-2-yl]carbonyl}-L-lysine, N⁶-[(Benzyloxy)carbonyl]lysine, (2*S*)-2-amino-6-[(cyclopentylcarbonyl)amino]hexanoic acid, N⁶-[(Cyclopentyloxy)carbonyl]-L-lysine, (2*S*)-2-amino-6-{[(2*R*)-tetrahydrofuran-2-ylcarbonyl]amino}hexanoic acid, (2*S*)-2-amino-8-[(2*R*,3*S*)-3-ethynyltetrahydrofuran-2-yl]-8-oxooctanoic acid, N⁶-(tert-Butoxycarbonyl)-L-lysine, (2S)-2-Hydroxy-6-({[(2-methyl-2-propanyl)oxy]carbonyl}amino)hexanoic acid, N⁶-[(Allyloxy)carbonyl]lysine, (2*S*)-2-amino-6-({[(2-azidobenzyl)oxy]carbonyl}amino)hexanoic acid, N⁶-L-Prolyl-L-lysine, (2*S*)-2-amino-6-{[(prop-2-yn-l-yloxy)carbonyl]amino}hexanoic acid and N*6*-[(2-Azidoethoxy)carbonyl]-L-lysine.

Preferably, the Pifl-like helicase further comprises: (a) substitution of at least one amino acid that interacts with one or more nucleotides in single-stranded DNA (ssDNA) or double-stranded DNA (dsDNA); and/or, (b) substitution of at least one amino acid that interacts with a transmembrane pore, wherein the Pifl-like helicase has the ability to control the movement of a polynucleotide.

More preferably, in (a), at least one amino acid that interacts with a sugar and/or base of one or more nucleotides in single-stranded DNA or double-stranded DNA is substituted with an amino acid containing a larger side chain (R group).

Preferably, the Pifl-like helicase comprises substitution of at least one amino acid that interacts with a sugar and/or base of one or more nucleotides in single-stranded DNA or double-stranded DNA with an amino acid containing a larger side chain (R group). Any number of amino acids may be substituted, such as, 1 or more, 2 or more, 3 or more, 4 or more, 5 or more, or 6 or more amino acids. Each amino acid may interact with a base, a sugar, or a base and a sugar. Protein modeling may be used to identify amino acids that interact with a sugar and/or base of one or more nucleotides in single-stranded or double-stranded DNA.

Preferably, the Pifl-like helicase comprises a variant of SEQ ID NO: 11, wherein the at least one amino acid that interacts with a sugar and/or base of one or more nucleotides in single-stranded DNA or double-stranded DNA is at least one of P73, H93, N99, F109, 1280, A161, F130, D132, D162, D163, E277, K415, Q291, H396, Y244 or P100. These numbers correspond to the relevant positions in SEQ ID NO: 11. Relative to SEQ ID NO: 11, it may be necessary to make changes when one or more amino acids have been inserted or deleted in the variant. As mentioned above, those skilled in the art may determine the corresponding positions in the variant. More preferably, the Pifl-like helicase comprises a variant of SEQ ID NO: 11, wherein the at least one amino acid that interacts with a sugar and/or base of one or more nucleotides in single-stranded DNA or double-stranded DNA is F109 and one or more of P73, H93, N99, F109, 1280, A161, F130, D132, D162, D163, E277, K415, Q291, H396, Y244 or P100.

Preferably, the Pifl-like helicase comprises a variant of any one of SEQ ID NOs: 1 to 10, wherein the at least one amino acid that interacts with a sugar and/or base of one or more nucleotides in single-stranded DNA or double-stranded DNA is at least one amino acid corresponding to P73, H93, N99, F109, 1280, A161, F130, D132, D162, D163, E277, K415, Q291, H396, Y244 or P100 in SEQ ID NO: 11. More preferably, the Pifl-like helicase comprises a variant of any one of SEQ ID NOs: 1 to 10, wherein the at least one amino acid that interacts with a sugar and/or base of one or more nucleotides in single-stranded DNA or double-stranded DNA is amino acid corresponding to F109 and one or more of amino acids corresponding to P73, H93, N99, F109, 1280, A161, F130, D132, D162, D163, E277, K415, Q291, H396, Y244 or P100 in SEQ ID NO: 11.

Table 4 shows amino acids in SEQ ID NOs:1 to 10 corresponding to P73, H93, N99, F109, 1280, A161, F130, D132, D162, D163, E277, K415, Q291, H396, Y244 and P100 in SEQ ID NO: 11.

**Table 4. Amino acid positions corresponding to SEQ ID NOs: 1 to 10**

| SEQ ID NO: | Amino acid positions | | | | | | | | | | | | | | |
|---|---|---|---|---|---|---|---|---|---|---|---|---|---|---|---|
| 11 | P73 | H93 | N99 | P100 | F109 | F130 | D132 | A161 | D162 | D163 | Y244 | E277 | 1280 | Q291 | H396 |
| 1 | P66 | H85 | N91 | P92 | F101 | M122 | G124 | V153 | E154 | P155 | F244 | E277 | 1281 | S291 | H401 |
| 2 | P65 | H85 | N92 | P92 | F101 | M122 | D124 | V153 | A154 | P155 | F245 | E278 | M281 | T292 | H399 |
| 3 | P65 | H85 | H91 | P92 | F101 | M126 | D128 | V157 | R158 | H159 | F246 | E279 | M282 | T293 | H410 |
| 4 | P65 | H85 | H91 | P92 | F101 | M126 | D128 | V157 | R158 | H159 | F246 | E279 | M282 | T293 | H411 |
| 5 | P63 | H82 | S88 | P89 | F98 | M119 | D121 | V150 | S151 | P152 | F240 | E273 | M276 | N287 | H398 |
| 6 | P67 | H88 | N93 | P94 | F103 | M124 | D126 | V155 | E156 | P157 | F246 | E279 | 1283 | S293 | H403 |
| 7 | P76 | H96 | N102 | P103 | F112 | M133 | D135 | V164 | A165 | P166 | F256 | E290 | M292 | T303 | H409 |
| 8 | P72 | H92 | S98 | P99 | F108 | M129 | D131 | V160 | S161 | P162 | Y254 | G287 | T291 | K301 | H411 |
| 9 | P63 | H82 | S88 | P89 | F98 | M119 | D121 | V150 | S151 | P152 | Y240 | E273 | M277 | N287 | H398 |
| 10 | P64 | H84 | N91 | P91 | F100 | M121 | D123 | V152 | E153 | L154 | Y245 | E278 | T281 | S292 | H400 |

The larger side chain (R group) preferably (a) comprises an increased number of carbon atoms, (b) has an increased length, (c) has an increased molecular volume, and/or (d) has an increased van der Waals volume. The larger side chain (R group) is preferably (a); (b); (c); (d); (a) and (b); (a) and (c); (a) and (d); (b) and (c); (b) and (d); (c) and (d); (a), (b) and (c); (a), (b) and (d); (a), (c) and (d); (b), (c) and (d); or (a), (b), (c) and (d). Each of (a) to (d) may be measured by standard methods in the art.

More preferably, the larger side chain (R group) increases (i) electrostatic interaction, (ii) hydrogen bonding and/or (iii) cation-pi (cation-π) interaction between the at least one amino acid and one or more nucleotides in the single-stranded DNA or double-stranded DNA. For example, in (i), positively charged amino acids such as arginine (R), histidine (H), and lysine (K) have R groups that increase electrostatic interaction. For example, in (ii), amino acids such as asparagine (N), serine (S), glutamine (Q), threonine (T), and histidine (H) have R groups that increase hydrogen bonding. For example, in (iii), aromatic amino acids such as phenylalanine (F), tryptophan (W), tyrosine (Y) or histidine (H) have R groups that increase cation-pi (cation-π) interaction.

The amino acid containing the larger side chain (R) may be non-natural amino acid. The non-natural amino acid may be any of those discussed below.

In a specific embodiment of the present application, the amino acid containing the larger side chain (R group) is not alanine (A), cysteine (C), glycine (G), selenocysteine (U), methionine (M), aspartic acid (D) or glutamic acid (E).

In a specific embodiment of the present application, the Pifl-like helicase comprises one or more of the following substitutions:
A) Histidine (H) is preferably substituted with (i) arginine (R) or lysine (K); (ii) glutamine (Q) or asparagine (N); or (iii) phenylalanine (F), tyrosine (Y) or tryptophan (W). Histidine (H) is more preferably substituted with (a) N, Q or W or (b) Y, F, Q or K.
B) Asparagine (N) is preferably substituted with (i) arginine (R) or lysine (K); (ii) glutamine (Q) or histidine (H); or (iii) phenylalanine (F), tyrosine (Y) or tryptophan (W). Asparagine (N) is more preferably substituted with R, H, W or Y.
C) Proline (P) is preferably substituted with (i) arginine (R) or lysine (K); (ii) glutamine (Q), asparagine (N), threonine (T) or histidine (H); (iii) tyrosine (Y), phenylalanine (F) or tryptophan (W); or (iv) leucine (L), valine (V) or isoleucine (I). Proline (P) is more preferably substituted with (i) arginine (R) or lysine (K); (ii) glutamine (Q), asparagine (N), threonine (T) or histidine (H); (iii) phenylalanine (F) or tryptophan (W) or (iv) leucine (L), valine (V) or isoleucine (I). Proline (P) is more preferably substituted with (a) F, (b) L, V, I, T or F or (c) W, F, Y, H, I, L or V.
D) Valine (V) is preferably substituted with (i) arginine (R) or lysine (K); (ii) glutamine (Q), asparagine (N) or histidine (H); (iii) phenylalanine (F), tyrosine (Y) or tryptophan (W); or (iv) isoleucine (I) or leucine (L). Valine (V) is more preferably substituted with (i) arginine (R) or lysine (K); (ii) glutamine (Q), asparagine (N) or histidine (H); (iii) tyrosine (Y) or tryptophan (W); or (iv) isoleucine (I) or leucine (L). Valine (V) is more preferably substituted with I or H or I, L, N, W or H.
E) Phenylalanine (F) is preferably substituted with (i) arginine (R) or lysine (K); (ii) histidine (H); or (iii) tyrosine (Y) or tryptophan (W). Phenylalanine (F) is more preferably substituted with (a) W, (b) W, Y or H, (c) W, R or K or (d) K, H, W or R.
F) Glutamine (Q) is preferably substituted with (i) arginine (R) or lysine (K) or (iii) phenylalanine (F), tyrosine (Y) or tryptophan (W).
G) Alanine (A) is preferably substituted with (i) arginine (R) or lysine (K); (ii) glutamine (Q), asparagine (N) or histidine (H); (iii) phenylalanine (F), tyrosine (Y) or tryptophan (W) or (iv) isoleucine (I) or leucine (L).
H) Serine (S) is preferably substituted with (i) arginine (R) or lysine (K); (ii) glutamine (Q), asparagine (N) or histidine (H); (iii) phenylalanine (F), tyrosine (Y) or tryptophan (W); or (iv) isoleucine (I) or leucine (L). Serine (S) is preferably substituted with K, R, W or F.
I) Lysine (K) is preferably substituted with (i) arginine (R) or (iii) tyrosine (Y) or tryptophan (W).
J) Arginine (R) is preferably substituted with (iii) Tyrosine (Y) or Tryptophan (W).
K) Methionine (M) is preferably substituted with (i) arginine (R) or lysine (K); (ii) glutamine (Q), asparagine (N) or histidine (H) or (iii) phenylalanine (F), tyrosine (Y) or tryptophan (W).
L) Leucine (L) is preferably substituted with (i) arginine (R) or lysine (K); (ii) glutamine (Q) or asparagine (N) or (iii) phenylalanine (F), tyrosine (Y) or tryptophan (W).
M) Aspartic acid (D) is preferably substituted with (i) arginine (R) or lysine (K); (ii) glutamine (Q), asparagine (N) or histidine (H); or (iii) phenylalanine (F), tyrosine (Y) or tryptophan (W). Aspartic acid (D) is more preferably substituted with H, Y or K.
N) Glutamic acid (E) is preferably substituted with (i) arginine (R) or lysine (K); (ii) glutamine (Q), asparagine (N) or histidine (H) or (iii) phenylalanine (F), tyrosine (Y) or tryptophan (W).
O) Isoleucine (I) is preferably substituted with (i) arginine (R) or lysine (K); (ii) glutamine (Q), asparagine (N) or histidine (H); (iii) phenylalanine (F), tyrosine (Y) or tryptophan (W) or (iv) leucine (L).
P) Tyrosine (Y) is preferably substituted with (i) arginine (R) or lysine (K); or (ii) tryptophan (W). Tyrosine (Y) is more preferably substituted with W or R.

In a specific embodiment of the present application, the Pifl-like helicase more preferably comprises a variant of SEQ ID NO: 11 comprising:

P73L; P73V; P73I; P73E; P73T; P73F; H93N; H93Q; H93W; N99R; N99H; N99W; N99Y; P100L; P100V; P100I; P100E; P100T; P100F; F130W; F130Y; F130H; D132H; D132Y; D132K; A161I; A161L; A161N; A161W; A161H; D162H; D162Y; D162K; D163W; D163F; D163Y; D163H; D163I; D163L; D163V; Y244W; Y244H; E277G; I280H; I280K; I280W; Q291K; Q291R; Q291W; Q291F; H396N; H396Q; H396W; K415W; K415R; K415H; K415Y; F109W/P73L; F109W/P73V; F109W/P73I; F109W/P73E; F109W/P73T; F109W/P73F; F109W/H93N; F109W/H93Q; F109W/H93W; F109W/N99R; F109W/N99H; F109W/N99W; F109W/N99Y; F109W/P100L; F109W/P100V; F109W/P1001; F109W/P100E; F109W/P100T; F109W/P100F; F109W/F130W; F109W/F130Y; F109W/F130H; F109W/D132H; F109W/D132Y; F109W/D132K; F109W/A161I; F109W/A161L; F109W/A161N; F109W/A161W; F109W/A161H; F109W/D162H; F109W/D162Y; F109W/D162K; F109W/D163W; F109W/D163F; F109W/D163Y; F109W/D163H; F109W/D163I; F109W/D163L; F109W/D163V; F109W/Y244W; F109W/Y244H; F109W/E277G; F109W/I280H; F109W/I280K; F109W/I280W; F109W/Q291K; F109W/Q291R; F109W/Q291W; F109W/Q291F; F109W/H396N; F109W/H396Q; F109W/H396W; F109W/K415W; F109W/K415R; F109W/K415H; or F109W/K415Y.

The helicase of the present application is preferably a helicase in which at least one amino acid that interacts with one or more phosphate groups of one or more nucleotides in ssDNA or dsDNA is substituted. Any number of amino acids may be substituted, for example, 1 or more, 2 or more, 3 or more, 4 or more, 5 or more, or 6 or more amino acids. The nucleotides in ssDNA each contain three phosphate groups. Each amino acid that is substituted may interact with any number of phosphate groups at a time, for example, one, two, or three phosphate groups at a time. Protein modeling may be used to identify amino acids that interact with one or more phosphate groups.

The substitution preferably increases (i) electrostatic interaction; (ii) hydrogen bonding and/or (iii) cation-pi (cation-π) interaction between the at least one amino acid and one or more phosphate groups in the ssDNA or dsDNA. The preferred substitutions of (i), (ii) and (iii) are discussed below using the labels (i), (ii) and (iii).

The substitution preferably increases the net positive charge of the position. Methods known in the art may be used to measure the net charge at any position. For example, the isoelectric point may be used to define the net charge of an amino acid. The net charge is usually measured at about 7.5. The substitution is preferably a substitution of a negatively charged amino acid with a positively charged, uncharged, non-polar or aromatic amino acid. A negatively charged amino acid is an amino acid with a net negative charge. Negatively charged amino acids include, but are not limited to, aspartic acid (D) and glutamic acid (E). A positively charged amino acid is an amino acid with a net positive charge. The positively charged amino acids may be naturally occurring or non-naturally occurring. The positively charged amino acids may be synthetic or modified. For example, modified amino acids with a net positive charge may be specifically designed for use in the present application. Many different types of modifications to amino acids are well known in the art. Preferred naturally occurring positively charged amino acids include, but are not limited to, histidine (H), lysine (K), and arginine (R).

Uncharged amino acids, non-polar amino acids, or aromatic amino acids may be naturally occurring or non-naturally occurring. It may be synthetic or modified. Uncharged amino acids have no net charge. Suitable uncharged amino acids include, but are not limited to, cysteine (C), serine (S), threonine (T), methionine (M), asparagine (N) and glutamine (Q). Non-polar amino acids have non-polar side chains. Suitable non-polar amino acids include, but are not limited to, glycine (G), alanine (A), proline (P), isoleucine (I), leucine (L) and valine (V). Aromatic amino acids have aromatic side chains. Suitable aromatic amino acids include, but are not limited to, histidine (H), phenylalanine (F), tryptophan (W), and tyrosine (Y).

The Pifl-like helicase preferably comprises a variant of SEQ ID NO: 11, in which the at least one amino acid that interacts with one or more phosphate groups of one or more nucleotides in ssDNA or dsDNA is at least one of H75, T91, S94, K97, N246, N247, N284, K288, N297, T394 or K397. These numbers correspond to the relevant positions in SEQ ID NO: 11. Relative to SEQ ID NO: 11, it may be necessary to make changes when one or more amino acids have been inserted or deleted in the variant. Those skilled in the art may determine the corresponding positions in the variants described above.

The Pifl-like helicase preferably comprises a variant of any one of SEQ ID NOs: 1 to 10, in which the at least one amino acid that interacts with one or more phosphate groups of one or more nucleotides in ssDNA or dsDNA is at least one amino acid corresponding to H75, T91, S94, K97, N246, N247, N284, K288, N297, T394 or K397 in SEQ ID NO: 11.

Table 5 shows the amino acids in SEQ ID NO: 1 to 10 corresponding to H75, T91, S94, K97, N246, N247, N284, K288, N297, T394, and K397 in SEQ ID NO: 11.

**Table 5. Amino acids positions in SEQ ID NOs: 1 to 10 corresponding to SEQ ID NO: 11.**

| SE Q ID NO: | Amino acids positions | | | | | | | | | | |
|---|---|---|---|---|---|---|---|---|---|---|---|
| 11 | H75 | T91 | S94 | K97 | N246 | N247 | N284 | K288 | N297 | T394 | K397 |
| 1 | H67 | T83 | R86 | K89 | N246 | K247 | E284 | K288 | N297 | T399 | K402 |
| 2 | H67 | T83 | S86 | K89 | N247 | K248 | K285 | K289 | N298 | T397 | K400 |
| 3 | H67 | T83 | S86 | K89 | N248 | D249 | E286 | E290 | N299 | T408 | K411 |
| 4 | H67 | T83 | S86 | K89 | N248 | D249 | E286 | E290 | N299 | T409 | K412 |
| 5 | H64 | T80 | S83 | K86 | N242 | K243 | E280 | K284 | N293 | T396 | K399 |
| 6 | H69 | T85 | R88 | K91 | N248 | K249 | E286 | K290 | N299 | T401 | K404 |
| 7 | H78 | T94 | S97 | K100 | N258 | K259 | T296 | K300 | N309 | T407 | K410 |
| 8 | H74 | T90 | S93 | K96 | N256 | K257 | T294 | K298 | N307 | T409 | K412 |
| 9 | H64 | T80 | S83 | K86 | N242 | K243 | E280 | K284 | N293 | T396 | K400 |
| 10 | H66 | T82 | S85 | K86 | N247 | E248 | K285 | K289 | N298 | T398 | K401 |

Preferably, the Pif1-like helicase comprises any one or more of the following:
a) Histidine (H) is substituted with (i) arginine (R) or lysine (K); (ii) asparagine (N), serine (S), glutamine (Q) or threonine (T); or (iii) phenylalanine (F), tryptophan (W) or tyrosine (Y);
b) Threonine (T) is substituted with (i) arginine (R), histidine (H) or lysine (K); (ii) asparagine (N), serine (S), glutamine (Q) or histidine (H); or (iii) phenylalanine (F), tryptophan (W), tyrosine (Y) or histidine (H);
c) Serine is substituted with (i) arginine (R), histidine (H) or lysine (K); (ii) asparagine (N), glutamine (Q), threonine (T) or histidine (H); or (iii) phenylalanine (F), tryptophan (W), tyrosine (Y) or histidine (H);
d) Asparagine (N) is substituted with (i) arginine (R), histidine (H) or lysine (K); (ii) serine (S), glutamine (Q), threonine (T) or histidine (H); or (iii) phenylalanine (F), tryptophan (W), tyrosine (Y) or histidine (H); and/or,
e) Lysine (K) is substituted with (i) arginine (R) or histidine (H); (ii) asparagine (N), serine (S), glutamine (Q), threonine (T) or histidine (H); or (iii) phenylalanine (F), tryptophan (W), tyrosine (Y) or histidine (H).

In a specific embodiment of the present application, the Pifl-like helicase is a variant of SEQ ID NO: 11 comprising one or more of (a) to (k): (a) H75N, H75Q, H75K or H75F; (b) T91K, T91Q or T91N; (c) S94H, S94N, S94K, S94T, S94R or S94Q; (d) K97Q, K97H or K97Y; (e) N246H or N246Q ; (f) N247Q or N247H; (g) N284H or N284Q; (h) K288Q or K288H; (i) N297Q, N297K or N297H; (j) T394K, T394H or T394N; or (k) K397R, K397H or K397Y.

The helicase of the present application is also such a helicase, wherein a part of the helicase that interacts with a transmembrane pore contains one or more modifications, preferably one or more substitutions. Further preferally, the helicase comprises substitution of at least one amino acid that interacts with a transmembrane pore. The part of the helicase that interacts with a transmembrane pore is usually the part of the helicase that interacts with the transmembrane pore when the helicase is used to control the movement of polynucleotides through the pore, for example, as discussed in more details below. When helicases are used to control the movement of polynucleotides through a pore, the part usually contains amino acids that interact or contact with the pore. When an electric potential is applied, and the helicase is bound or linked to a polynucleotide that is moving through the pore, the part usually contains amino acids that interact or contact with the pore.

The part that interacts with the transmembrane pore preferably comprises a variant of SEQ ID NO: 11, wherein one or more, such as 2, 3, 4 or 5 amino acids on E196, W202, N199 or G201 are substituted.

The part that interacts with a transmembrane pore preferably comprises a variant of any one of SEQ ID NOs: 1 to 10, which comprises substitutions of at least one or more of the amino acids corresponding to SEQ ID NO: 11 at positions (a) E196; (b) W202; (c); N199; or (d) G201.

Table 6 shows amino acids in SEQ ID NOs: 1 to 10 corresponding to E196, W202, N199, and G201 in SEQ ID NO: 11.

**Table 6. Amino acid positions in SEQ ID NOs: 1 to 10 corresponding to SEQ ID NO: 11**

| SEQIDNO: | Amino acid positions | | | |
|---|---|---|---|---|
| 11 | E196 | W202 | N199 | G201 |
| 1 | E193 | W199 | M196 | S198 |
| 2 | E192 | W198 | H195 | K197 |
| 3 | K196 | W202 | N199 | G201 |
| 4 | K196 | W202 | N199 | G201 |
| 5 | E189 | W195 | N192 | G194 |
| 6 | D195 | W201 | T198 | G200 |
| 7 | E203 | W209 | K206 | G208 |
| 8 | E199 | W205 | K202 | Q204 |
| 9 | E189 | W195 | T192 | G194 |
| 10 | S192 | W198 | T195 | G197 |

In a specific embodiment of the present application, the Pifl-like helicase is a variant of SEQ ID NO. 11 comprising substitutions at the following positions:
- F109/E196/H75, such as, F109W/E196L/H75N, F109W/E196L/H75Q, F109W/E196L/H75K or F109W/E196L/H75F;
- F109/E196/T91, such as, F109W/E196L/T91K, F109W/E196L/T91Q or F109W/E196L/T91N;
- F109/S94/E196, such as, F109W/S94H/E196L, F109W/S94T/E196L, F109W/S94R/E196L, F109W/S94Q/E196L, F109W/S94N/E196L, or F109W/S94K/E196L;
- F109/N99/E196, such as, F109W/N99R/E196L, F109W/N99H/E196L, F109W/N99W/E 196L or F109W/N99Y/E196L;
- F109/S94/E196/I280, such as, F109W/S94H/E196L/I280K;
- F109/P100/E196, such as, F109W/P100L/E196L, F109W/P100V/E196L, F109W/P100I/E196L or
- F109W/P100T/E196L;
- F109/D132/E196, such as, F109W/D132H/E196L, F109W/D132Y/E196L or F109W/D132K/E196L;
- F109/A161/E196, such as, F109W/A161I/E196L, F109W/A161L/E196L, F109W/A161N/E196L, F109W/A161W/E196L or F109W/A161H/E196L;
- F109/D163/E196, such as, F109W/D163W/E196L, F109W/D163F/E196L, F109W/D163Y/E196L, F109W/D163H/E196L, F109W/D163I/E196L, F109W/D163L/E196L or F109W/D163V/D163L/E196L;
- F109/Y244/E196, such as, F109W/Y244W/E196L, F109W/Y244Y/E196L or F109W/Y244H/E196L;
- F109/N246/E196, such as, F109W/N246H/E196L or F109W/N246Q/E196L;
- F109/E196/I280, such as, F109W/E196L/I280K, F109W/E196L/I280H, F109W/E196L/I280W or F109W/E196L/I280R;
- F109/E196/Q291, such as, F109W/E196L/Q291K, F109W/E196L/Q291R, F109W/E196L/Q291W or F109W/E196L/Q29IF;
- F109/N297/E196, such as, F109W/N297Q/E196L, F109W/N297K/E196L or F109W/N297H/E196L;
- F109/T394/E196, such as, F109W/T394K/E196L, F109W/T394H/E196L or F109W/T394N/E196L;
- F109/H396/E196, such as, F109W/H396Y/E196L, F109W/H396F/E196L, F109W/H396Q/E196L or F109W/H396K/E196L;
- F109/K397/E196, such as, F109W/K397R/E196L, F109W/K397H/E196L or F109W/K397Y/E196L; or,
- F109/Y416/E196, such as, F109W/Y416W/E196L or F109W/Y416R/E196L.

A variant of a Pif1-like helicase is an enzyme that has an amino acid sequence which varies from that of the wild-type helicase and which retains polynucleotide binding activity. In particular, a variant of any one of SEQ ID NOs: 1 to 11 is an enzyme that has an amino acid sequence which varies from that of any one of SEQ ID NOs: 1 to 11 and which retains polynucleotide binding activity. Polynucleotide binding activity can be determined using methods known in the art. Suitable methods include, but are not limited to, fluorescence anisotropy, tryptophan fluorescence and electrophoretic mobility shift assay (EMSA). For instance, the ability of a variant to bind a single stranded polynucleotide can be determined as described in the Examples.

Over the entire length of the amino acid sequence of any one of SEQ ID NOs: 1 to 11, a variant will preferably be at least 20% homologous to that sequence based on amino acid identity. More preferably, the variant polypeptide may be at least 30%, at least 40%, at least 45%), at least 50%, at least 55%, at least 60%, at least 65%, at least 70%, at least 75%, at least 80%, at least 85%, at least 90% and more preferably at least 95%, 97% or 99% homologous based on amino acid identity to the amino acid sequence of any one of SEQ ID NOs: 1 to 11 over the entire sequence. There may be at least 70%, for example at least 80%, at least 85%, at least 90% or at least 95%, amino acid identity over a stretch of 100 or more, for example 150, 200, 300, 400 or 500 or more, contiguous amino acids ("strict homology").

In the second aspect of the present application, provided is a construct, which comprises at least one or more Pif1-like helicases of the present application.

Preferably, the construct further comprises a polynucleotide binding moiety.

Preferably, the construct has the ability to control the movement of a polynucleotide.

Preferably, the polynucleotide binding moiety may be a moiety that binds to a base of the polynucleotide, and/or a moiety that binds to a sugar of the polynucleotide, and/or a moiety that binds to a phosphate group of the polynucleotide.

Preferably, the Pifl-like helicase and polynucleotide binding moiety constituting the construct can be prepared separately and then directly connected. The construct can also be directly prepared by genetic fusion. For example, the nucleotides encoding the Pifl-like helicase and the polynucleotide binding moiety are connected, then transferred into host cells for expression, and purifed.

More preferably, the polynucleotide binding moiety is a polypeptide capable of binding to a polynucleotide, including but not limited to one or a combination of two or more of eukaryotic single-chain binding proteins, bacterial single-chain binding proteins, archaea single-chain binding proteins, and viral single-chain binding proteins or double-stranded binding proteins.

In a specific embodiment of the present application, the polynucleotide binding moiety includes but is not limited to any one of those shown in Table 7:

**Table 7. Binding moiety bound to a polynucleotide**

| Name | Classification | Source | UniProt Database | Molecular Weight (Da) |
|---|---|---|---|---|
| SSBEco | ssb | Escherichia coli | P0AGE0 | 18975 |
| SSBBhe | ssb | Bartonella henselae | Q6G302 | 19312 |
| SSBCbu | ssb | Coxiella burnetii | Q83EP4 | 17437 |
| SSBTma | ssb | Thermathogamaritima | Q9WZ73 | 16298 |
| SSBHpy | ssb | Helicobacter pylori | 025841 | 20043 |
| SSBDra | ssb | Deinococcusradiodurans | Q9RY51 | 32722 |
| SSBTaq | ssb | Thermus aquaticus | Q9KH06 | 30029 |
| SSBMsm | ssb | Mycobacterium smegmatis | Q9AFI5 | 17401 |
| SSBSso | ssb/RPA | Sulfolobussolfataricus | Q97W73 | 16138 |
| SSBSso7D | ssb | Sulfolobussolfataricus | P39476 | 7280 |
| SSBMHsmt | ssb | Homo sapiens | Q04837 | 17260 |
| SSBMle | ssb | Mycobacterium leprae | P46390 | 17701 |
| gp32T4 | ssb | Bacteriohage T4 | P03695 | 33506 |
| gp32RB69 | ssb | Bacteriophage RB69 | Q7Y265 | 33118 |
| gp2.5T7 | ssb | Bacteriohage T7 | P03696 | 25694 |

In the third aspect of the present application, provided is a nucleic acid that encodes the Pifl-like helicase or the construct of the present application.

In the fourth aspect of the present application, provided is an expression vector comprising the nucleic acid of the present application.

Preferably, the nucleic acid is operably linked to a regulatory element in an expression vector, wherein the regulatory element is preferably a promoter.

In a specific embodiment of the present application, the promoter is selected from T7, trc, lac, ara or λ_{L}.

Preferably, the expression vector includes but is not limited to a plasmid, virus or phage.

In the fifth aspect of the present application, provided is a host cell, comprising the nucleic acid or the expression vector of the present application.

Preferably, the host cell includes but is not limited to *Escherichia coli.*

In a specific embodiment of the present application, the host cell is selected from BL21 (DE3), JM109 (DE3), B834 (DE3), TUNER, C41 (DE3), Rosetta2 (DE3), Origami, Origami B, and the like.

In the sixth aspect of the present application, provided is a method for preparing Pifl-like helicase. The method comprises providing a wild-type Pifl-like helicase, and then modifying the wild-type Pifl-like helicase to obtain the Pifl-like helicase of the present application.

In the seventh aspect of the present application, provided is a method for preparing Pifl-like helicase. The method comprises culturing the host cell of the present application, and performing an inducible expression and purification to obtain the Pifl-like helicase.

In a specific embodiment of the present application, the method comprises obtaining a nucleic acid sequence encoding a Pifl-like helicase according to the amino acid sequence of the Pifl-like helicase of the present application, digesting the nucleic acid sequence and ligating to a expression vector, then transforming into *E. coli*, and performing an inducible expression and purification to obtain the Pifl-like helicase.

In the eighth aspect of the present application, provided is a method of controlling the movement of a polynucleotide, comprising contacting the Pifl-like helicase or the construct of the present application with the polynucleotide.

Preferably, said controlling the movement of the polynucleotide is controlling the movement of the polynucleotide through a pore. The pore is a nanopore, and the nanopore is a transmembrane pore. The pore may be a natural or artificial pore, including but not limited to a protein pore, a polynucleotide pore or a solid state pore.

In a specific embodiment of the present application, the transmembrane pore is selected from a biological pore, a solid state pore, or a biological and solid state hybrid pore.

In a specific embodiment of the present application, the pores include, but are not limited to, those derived from Mycobacterium smegmatis porin A, Mycobacterium smegmatis porin B, Mycobacterium smegmatis porin C, and Mycobacterium smegmatis porin D, hemolysin, lysin, interleukin, outer membrane porin F, outer membrane porin G, outer membrane phospholipase A, WZA or Neisseria autotransporter lipoprotein, etc.

Preferably, the method may include controlling the movement of the polynucleotide with one or more Pifl-like helicases.

In the ninth aspect of the present application, provided is a method for characterising a target polynucleotide, comprising:
I) contacting the Pifl-like helicase or the construct of the present application with the target polynucleotide and a pore, such that the Pifl-like helicase or the construct controls the movement of the target polynucleotide through the pore; and
II) taking one or more characteristics of the target polynucleotide when the nucleotide of the target polynucleotide interacts with the pore, and thereby characterising the target polynucleotide.

Any number of Pifl-like helicases of the present application may be used in these methods. More preferably, 1, 2, 3, 4, 5, 6, 7, 8, 9, 10 or more helicases may be used. If two or more Pifl-like helicases of the present application are used, they may be the same or different. It may also comprise wild-type Pifl-like helicases or other types of helicases. Further, if two or more helicases are used, they may be attached to one another, or bound to the polynucleotide separately to perform the function of controlling the movement of the polynucleotide.

Preferably, when a force (such as a voltage) is applied to the pore, speed of the polynucleotide passing through the pore is controlled by the Pifl-like helicase or construct, so as to obtain a recognizable and stable current level for determining the characteristics of the target polynucleotide.

Preferably, steps I) and II) are repeated one or more times.

Preferably, the method further comprises the step of applying a potential difference across the pore contacting the helicase or the construct and the target polynucleotide.

Preferably, the pore is a structure that permits hydrated ions driven by an applied potential to flow from one side of the membrane to the other side of the membrane. More preferably, the pores are nanopores, and the nanopores are transmembrane pores. The transmembrane pores provide channels for the movement of the target polynucleotide.

The membrane may be any membranes well-known in the art, perpfrally an amphiphilic layer, i.e., a layer formed from amphiphilic molecules, such as phospholipids, which have both at least one hydrophilic portion and at least one lipophilic or hydrophobic portion. The amphiphilic molecules may be synthetic or naturally occurring. More preferably, the membrane is a lipid bilayer membrane.

The target polynucleotide may be coupled to the membrane using any known method. If the membrane is an amphiphilic layer, such as a lipid bilayer, the polynucleotide is preferably coupled to the membrane via a polypeptide present in the membrane or a hydrophobic anchor present in the membrane. The hydrophobic anchor is preferably a lipid, fatty acid, sterol, carbon nanotube or amino acid.

Preferably, the pore is selected from a biological pore, a solid state pore, or a biological and solid state hybrid pore.

In a specific embodiment of the present application, the pores include, but are not limited to, those derived from Mycobacterium smegmatis porin A, Mycobacterium smegmatis porin B, Mycobacterium smegmatis porin C, and Mycobacterium smegmatis porin D, hemolysin, lysin, interleukin, outer membrane porin F, outer membrane porin G, outer membrane phospholipase A, WZA or Neisseria autotransporter lipoprotein, etc.

When provided with all the necessary components to facilitate movement, the Pif1-like helicase moves along the DNA in the 5'-3' direction, but the orientation of the DNA in the nanopore (dependent on which end of the DNA is captured) means that the enzyme can be used to either move the DNA out of the nanopore against the applied field, or move the DNA into the nanopore with the applied field.

Preferably, the target polynucleotide is single-stranded, double-stranded, or at least partially double-stranded.

More preferably, the target polynucleotide may be modified by tags, spacers, methylation, oxidation or damage.

In a specific embodiment of the present application, at least a part of the target polynucleotide is double-stranded. The double-stranded part constitutes a Y adapter structure comprising a leader sequence that is preferentially screwed into the pore.

More preferably, the length of the target polynucleotide may be 10 to 100000 or more.

In a specific embodiment of the present application, the length of the target polynucleotide may be at least 10, at least 50, at least 100, at least 200, at least 300, at least 400, at least 500, at least 1,000, at least 2,000, at least 5,000, at least 10,000, at least 50,000, or at least 100,000, etc.

Preferably, the helicase is incorporated into an internal nucleotide of a single-stranded polynucleotide.

Preferably, the one or more characteristics are selected from the source, length, identity, sequence, secondary structure of the target polynucleotide, or whether or not the target polynucleotide is modified.

Preferably, taking the one or more characteristics are carried out by electrical measurement and/or optical measurement.

More preferably, electrical and/or optical signals are generated by electrical measurement and/or optical measurement, wherein each nucleotide corresponds to a signal level, and then the electrical signals and/or optical signals are converted into characteristics of the nucleotide.

In a specific embodiment of the present application, the electrical measurement includes, but is not limited to, a current measurement, an impedance measurement, a tunnelling measurement, a wind tunnel measurement or a field effect transistor (FET) measurement, etc.

The electrical signal of the present application is selected from the measured values of current, voltage, tunneling, resistance, potential, conductivity or lateral electrical measurement.

In a specific embodiment of the present application, the electrical signal is a current passing through the pore.

Preferably, the characterising further comprises using an improved Viterbi algorithm.

In the tenth aspect of the present application, provided is a product for characterising a target polynucleotide, comprising the Pifl-like helicase, the construct, the nucleic acid, the expression vector or the host cell of the present application, and a pore.

Preferably, the product comprises multiple Pifl-like helicases and/or multiple constructs.

Preferably, the product comprises multiple pores. More preferably, the pores are nanopores, and the nanopores are transmembrane pores.

In a specific embodiment of the present application, the transmembrane pore is selected from a biological pore, a solid state pore, or a biological and solid state hybrid pore.

In a specific embodiment of the present application, the pores include, but are not limited to, those derived from Mycobacterium smegmatis porin A, Mycobacterium smegmatis porin B, Mycobacterium smegmatis porin C, and Mycobacterium smegmatis porin D, hemolysin, lysin, interleukin, outer membrane porin F, outer membrane porin G, outer membrane phospholipase A, WZA or Neisseria autotransporter lipoprotein, etc.

In a specific embodiment of the present application, the product comprises multiple Pifl-like helicases or multiple constructs, and multiple pores.

Preferably, the product is selected from a kit, a device or a sensor.

More preferably, the kit also comprises a chip containing a lipid bilayer. The pores span the lipid bilayer.

The kit of the present application comprises one or more lipid bilayers, and each lipid bilayer comprises one or more of the pores.

The kit of the present application also comprises reagents or devices for performing the characterising of a target polynucleotide. Preferably, the reagents include buffers and tools required for PCR amplification.

In the eleventh aspect of the present application, provided is use of the Pif1-like helicase, the construct, the nucleic acid, the expression vector, the host cell or the product of the present application in characterising a target polynucleotide or controlling the movement of a target polynucleotide through a pore.

In the twelfth aspect of the present application, provided is a kit for characterising a target polynucleotide, comprising the Pifl-like helicase, the construct or the nucleic acid, the expression vector or the host cell of the present application, and pores.

In the thirteenth aspect of the present application, provided is a device for characterising a target polynucleotide, comprising the Pifl-like helicase, the construct or the nucleic acid, the expression vector or the host cell of the present application, and the pore.

Preferably, the device comprises a sensor that is capable of supporting the plurality of pores and can transmit signals of the interaction of the pores with the polynucleotide, and at least one reservoir for storing the target polynucleotide, and a solution required to perform the characterising.

Preferably, the device comprises a plurality of Pifl-like helicases and/or a plurality of constructs, and a plurality of pores.

In the fourteenth aspect of the present application, provided is a sensor for characterising a target polynucleotide, comprising a complex formed between the pore and the Pifl-like helicase or the construct of the present application.

Preferably, the pore is brought into contact with the helicase or construct in the presence of the target polynucleotide, and a potential is applied across the pore. The potential is selected from voltage potential or chemical potential.

In the fifteenth aspect of the present application, provided is a method for forming a sensor for characterising a target polynucleotide, comprising forming a complex between the pore and the Pifl-like helicase or the construct of the present application , thereby forming a sensor that characterises the target polynucleotide.

In the sixteenth aspect of the present application, provided is a structure of two or more helicases attached to a polynucleotide, wherein at least one of the two or more helicases is a Pifl-like helicase of the present application.

In the seventeenth aspect of the present application, provided is a Pifl-like helicase oligomer. The Pifl-like helicase oligomer comprises one or more Pifl-like helicases of the present.

Preferably, the Pifl-like helicase oligomer may also comprise wild-type Pifl-like helicase or other types of helicase, wherein the other types of helicase may be Hel308 helicase, XPD helicase, Dda helicase, TraI helicase or TrwC helicase, etc.

Preferably, the Pifl-like helicase and the wild-type Pifl-like helicase, the Pifl-like helicase and the Pifl-like helicase, the wild-type Pifl-like helicase and the wild-type Pifl-like helicase, the Pifl-like helicase and other types of helicases, or the wild-type Pifl-like helicase and other types of helicases, may be connected or arranged in a head-to-head, tail-to-tail or head-to-tail manner.

Preferably, the Pifl-like helicase oligomer comprises more than two Pifl-like helicases of the present application, wherein the Pifl-like helicases may be different or the same.

The "Pif1-like helicase" of the present application is modified relative to the wild-type or natural helicase.

The "Pif1-like helicase", "construct" or "pore" of the present application may be modified to assist their identification or purification, for example by the addition of histidine residues (a his tag), aspartic acid residues (an asp tag), a streptavidin tag, a Flag tag, a SUMO tag, a GST tag or a MBP tag, or by the addition of a signal sequence to promote their secretion from a cell where the polypeptide does not naturally contain such a sequence. An alternative to introducing a genetic tag is to chemically react a tag onto a native or engineered position on the helicase, pore or construct.

The "nucleotides" in the present application include, but are not limited to, adenosine monophosphate (AMP), guanosine monophosphate (GMP), thymidine monophosphate (TMP), uridine monophosphate (UMP), cytidine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP) and deoxycytidine monophosphate (dCMP). The nucleotides are preferably selected from AMP, TMP, GMP, CMP, UMP, dAMP, dTMP, dGMP, dCMP and dUMP.

The "conservative amino acid substitutions" in the present application include, but are not limited to, a substitution between alanine and serine, glycine, threonine, valine, proline or glutamic acid; and/or a substitution between aspartame and glycine, asparagine or glutamic acid; and/or a substitution between serine and glycine, asparagine or threonine; and/or a substitution between leucine and isoleucine or valine; and/or a substitution between valine and leucine, isoleucine; and/or a substitution between tyrosine and phenylalanine; and/or, a substitution between lysine and arginine. The above-mentioned substitution basically does not change the activity of the amino acid sequence of the present application.

The term "two or more" in the present application includes two, three, four, five, six, seven, eight or more and so on.

The term "plurality"or "multiple" in the present application includes but is not limited to two or more, three or more, four or more, five or more, six or more, seven or more, eight or more or more, and so on.

The term "at least one" in the present application includes but is not limited to one or more, two or more, three or more, four or more, five or more, six or more, seven or more, eight or more or more, and so on.

The term "and/or" in the present application includes the selection of one and any number of combinations of the listed items.

The term "comprise/comprising" in the present application is an open-ended term, comprising the specified ingredients or steps described, as well as other ingredients or steps that have no substantial effect.

The term "non-natural amino acid" in the present application is an amino acid that is not naturally present in Pif1-like helicase. Preferably, it includes but is not limited to, 4-Azido-L-phenylalanine (Faz), 4-Acetyl-L-phenylalanine, 3-Acetyl-L-phenylalanine, 4-Acetoacetyl-L-phenylalanine, O-Allyl-L-tyrosine, 3-(Phenylselanyl)-L-alanine, O-2-Propyn-l-yl-L-tyrosine, 4-(Dihydroxyboryl)-L-phenylalanine, 4-[(Ethylsulfanyl)carbonyl]-L-phenylalanine, (2*S*)-2-amino-3-{4-[(propan-2-ylsulfanyl)carbonyl]phenyl}propanoic acid, (2*S*)-2-amino-3-{4-[(2-amino-3-sulfanylpropanoyl)amino]phenyl}propanoic acid, O-Methyl-L-tyrosine, 4-Amino-L-phenylalanine, 4-Cyano-L-phenylalanine, 3-Cyano-L-phenylalanine, 4-Fluoro-L-phenylalanine, 4-Iodo-L-phenylalanine, 4-Bromo-L-phenylalanine, O-(Trifluoromethyl)tyrosine, 4-Nitro-L-phenylalanine, 3-Hydroxy-L-tyrosine, 3-Amino-L-tyrosine, 3-Iodo-L-tyrosine, 4-Isopropyl-L-phenylalanine, 3-(2-Naphthyl)-L-alanine, 4-Phenyl-L-phenylalanine, (2*S*)-2-amino-3-(naphthalen-2-ylamino)propanoic acid, 6-(Methylsulfanyl)norleucine, 6-Oxo-L-lysine, D-tyrosine, (2R)-2-Hydroxy-3-(4-hydroxyphenyl)propanoic acid, (2R)-2-Ammoniooctanoate3-(2,2'-Bipyridin-5-yl)-D-alanine, 2-amino-3-(8-hydroxy-3-quinolyl)propanoic acid, 4-Benzoyl-L-phenylalanine, S-(2-Nitrobenzyl)cysteine, (2R)-2-amino-3-[(2-nitrobenzyl)sulfanyl]propanoic acid, (2*S*)-2-amino-3-[(2-nitrobenzyl)oxy]propanoic acid, O-(4,5-Dimethoxy-2-nitrobenzyl)-L-serine, (2S)-2-amino-6-({[(2-nitrobenzyl)oxy]carbonyl}amino)hexanoic acid, O-(2-Nitrobenzyl)-L-tyrosine, 2-Nitrophenylalanine, 4-[(E)-Phenyldiazenyl]-L-phenylalanine, 4-[3-(Trifluoromethyl)-3H-diaziren-3-yl]-D-phenylalanine, 2-amino-3-[[5-(dimethylamino)-l-naphthyl]sulfonylamino]propanoic acid, (2*S*)-2-amino-4-(7-hydroxy-2-oxo-2H-chromen-4-yl)butanoic acid, (2*S*)-3-[(6-acetylnaphthalen-2-yl)amino]-2-aminopropanoic acid, 4-(Carboxymethyl)phenylalanine, 3-Nitro-L-tyrosine, O-Sulfo-L-tyrosine, (2R)-6-Acetamido-2-ammoniohexanoate, 1-Methylhistidine, 2-Aminononanoic acid, 2-Aminodecanoic acid, L-Homocysteine, 5-Sulfanylnorvaline, 6-Sulfanyl-L-norleucine, 5-(Methyl sulfanyl)-L-norvaline, N⁶-{[(2R,3R)-3-Methyl-3,4-dihydro-2H-pyrrol-2-yl]carbonyl}-L-lysine, N⁶-[(Benzyloxy)carbonyl]lysine, (2*S*)-2-amino-6-[(cyclopentylcarbonyl)amino]hexanoic acid, N⁶-[(Cyclopentyloxy)carbonyl]-L-lysine, (2*S*)-2-amino-6-{[(2*R*)-tetrahydrofuran-2-ylcarbonyl]amino}hexanoic acid, (2*S*)-2-amino-8-[(2*R*,3*S*)-3-ethynyltetrahydrofuran-2-yl]-8-oxooctanoic acid, N⁶-(tert-Butoxycarbonyl)-L-lysine, (2S)-2-Hydroxy-6-({[(2-methyl-2-propanyl)oxy]carbonyl}amino)hexanoic acid, N⁶-[(Allyloxy)carbonyl]lysine, (2*S*)-2-amino-6-({[(2-azidobenzyl)oxy]carbonyl}amino)hexanoic acid, N⁶-L-Prolyl-L-lysine, (2*S*)-2-amino-6-{[(prop-2-yn-l-yloxy)carbonyl]amino}hexanoic acid and N*6*-[(2-Azidoethoxy)carbonyl]-L-lysine.

### BRIEF DESCRIPTION OF THE DRAWINGS

Hereinafter, the embodiments of the present application will be described in detail with reference to the accompanying drawings, in which:

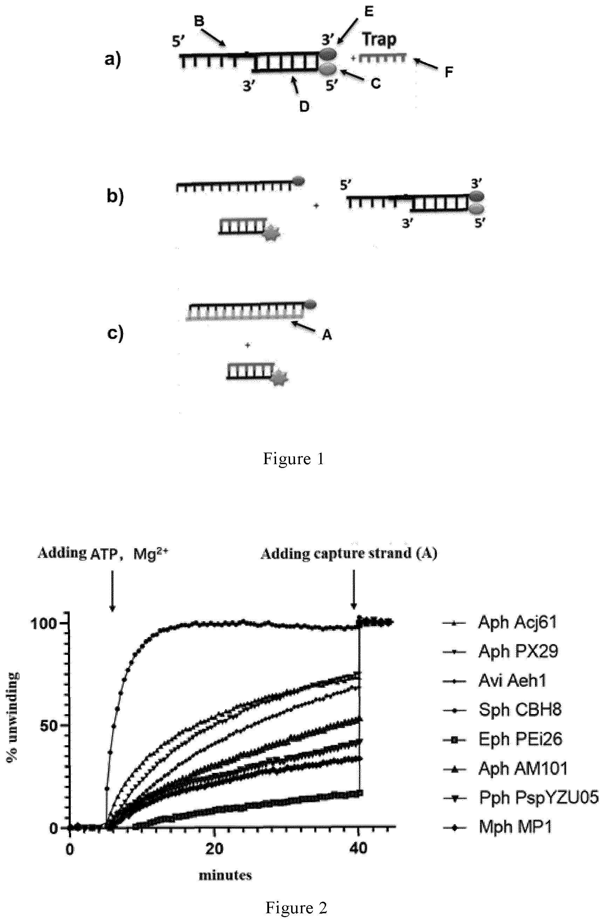

Figure 1 shows a schematic diagram of fluorescence assay for testing enzyme activity of Pifl-like helicase, wherein the fluorescent substrate strand has a 5' ssDNA overhang, and a 50 base section of hybridized dsDNA. As shown in a), the major upper strand (B) has a black-hole quencher (BHQ-1) base (E) at the 3' end, and the hybridized complement (D) has a carboxyfluorescein base (C) at the 5' end. 0.5 µM of a capture strand (F) that is complementary to the shorter strand of the fluorescent substrate (D) is also included. As shown in b), in the presence of ATP (5 mM) and MgCl₂ (5 mM), helicase (200 nM) added to the substrate binds to the 5' part of the fluorescent substrate, moves along the major strand, and unwinds the complementary strand. After that, excess of capture strand preferentially anneals to the complementary DNA to prevent re-annealing of initial substrate and loss of fluorescence. As shown in c), after adding the excess of the capture strand (A) that is completely complementary to the major strand, part of the entangled dsDNA will have a strand unwinding effect due to the presence of excessive A, and finally all the dsDNA will be untwisted, and the fluorescence value will reach the highest.
Figure 2 shows a measurement of the hybridized dsDNA unwinding capabilities of Pifl-like helicase using a fluorescence assay, specifically a graph of the time-dependent dsDNA unwinding ratio in a buffer containing 400mM NaCl.

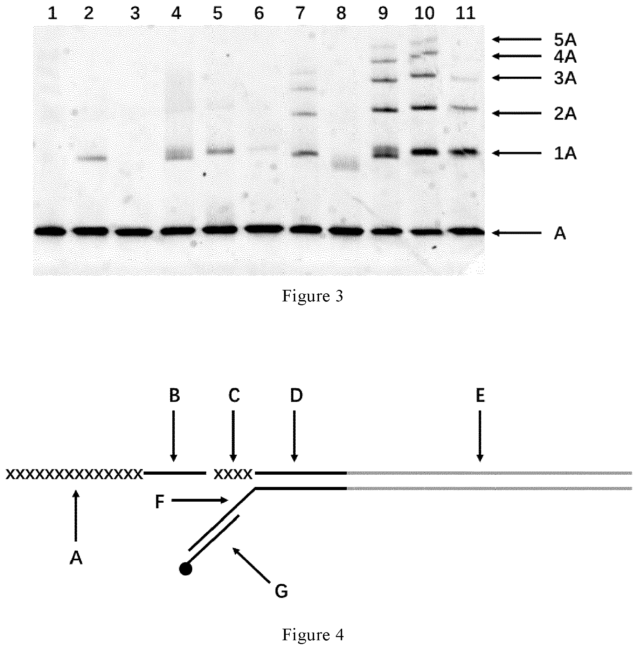

Figure 3 shows gel measurements of the DNA-binding capabilities of different PIF1-like helicases. Lane 1 represents the pre-constructed dsDNA (hybridization of SEQ ID NO: 18 with SEQ ID NO: 19 modified with 5'FAM). Lanes 2-6 comprise Aph Acj61, Aph PX29, Sph CBH8, PphPspYZU05, and Mph MP1 pre-linked to dsDNA, respectively. Lanes 7-11 comprise Aph Acj61-D97C/A363C, Aph PX29-D96C/A371C, Sph CBH8-A94C/A361C/C136A, PphPspYZU05-D104C/A375C/C146A and Mph MP1-E105C/A362C pre-linked to dsDNA, respectively. Band A corresponds to SEQ ID NO:19 to be hybridized with SEQ ID NO:18. The regions labeled 1A, 2A, 3A, 4A, and 5A correspond to combination of 1, 2, 3, 4, and 5 helicases with hybridization of SEQ ID NO: 18 with SEQ ID NO: 19, respectively.
Figure 4 shows DNA construct which is used in examples, in which SEQ ID NO: 13 (labelled B) is attached at its 5' end to twenty iSpC3 spacers (labelled A) and at its 3' end to four iSpC3 spacers (labelled C) which are attached to the 5' end of SEQ ID NO: 14 (labelled D) which is attached at its 3' end to SEQ ID NO: 17 or 24 (labelled E), and the SEQ ID NO: 15 region (labelled F) of this construct is hybridized to SEQ ID NO: 16 (labelled G, which has a 3' cholesterol tether).
Figure 5 shows an illustrative current trace (y-axis = Current (pA, 0 to 250), x-axis = Sampling frequency (hz, 0 to 3.5^{∗}10⁵)) when Pifl-like helicase (Mph MP1-E105C/A362C (SEQ ID NO: 11 with mutations E105C/A362C)) controlled the translocation of DNA construct A through the nanopore 8MspA (SEQ ID NO: 12).
Figure 6 shows an enlarged current trace in regions of the Pifl-like helicase-controlled DNA movement shown in Figure 5 (y-axis = Current (pA, 30 to 100, x-axis = Sampling frequency (hz, 2.346 to 2.366^{∗}10⁵)).
Figure 7 shows an illustrative current trace (y-axis = Current (pA), x-axis = Time (s)) when Pifl-like helicase (Sph CBH8-A94C/A361C/C136A (SEQ ID NO: 5 with A94C/A361C/C136A mutation)) controlled the translocation of DNA construct B through the MspA nanopore.
Figure 8 shows an illustrative current trace (y-axis = Current (pA), x-axis = Time (s)) when helicase (Eph Pei26-D99C/A366C/C141A (SEQ ID NO: 6 with D99C/A366C/C141A mutation)) controlled the translocation of DNA construct B through the MspA nanopore.
Figure 9 shows an illustrative current trace (y-axis = Current (pA), x-axis = Time (s)) when helicase (Pph PspYZU05-D104C/A375C/C146A (SEQ ID NO: 8 with D104C/A375C and C146A mutations)) controlled the translocation of DNA construct B through the MspA nanopore.
Figure 10 shows the SDS-PAGE gel electrophoresis image of purified Mph MP1 (SEQ ID NO: 11), in which M represents Marker (Kd), and lane 1 represents the electrophoresis result of the helicase Mph MP1.
Figure 11 shows the correspondence among the amino acid sequences of SEQ NOs: 1 to 11.

### DETAILED DESCRIPTION

The following examples further illustrate the content of the present application, but should not be construed as limiting the present application. Without departing from the spirit and essence of the present application, modifications or substitutions made to the methods, steps or conditions of the present application fall within the scope of claims.

Unless otherwise specified, the technical means used in the examples are conventional means well known to those skilled in the art. The equipment and reagents used in each example are conventionally commercially available.

### Example 1. Preparation of Pifl-like helicase

### 1. Materials and methods

A recombinant plasmid containing the Pifl-like helicase sequence (a variant of amino acid sequence SEQ ID NOs: 1-11, corresponding to nucleotide sequence SEQ ID NOs:25-35) was transformed into BL21(DE3) competent cell by heat shock. The resuscitated bacteria solution was spread on an ampicillin-resistant solid LB plate, and then cultured overnight at 37°C. A single colony was picked and inoculated into 100ml of ampicillin-resistance liquid LB medium and cultivated at 37°C. 1% of the inoculum was transferred to ampicillin-resistant LB liquid medium for expansion culture at 37°C and 200 rpm, and its OD600 value was measured continuously. When OD600=0.6-0.8, the culture solution in LB medium was cooled to 18°C, and isopropyl thiogalactoside (Isopropyl β-D-Thiogalactoside, IPTG) was added to induce expression, so that the final concentration reached 1 mM. After 12-16h, the bacteria were collected at 18°C. The bacteria were crushed under high pressure, purified by FPLC, and samples were collected.

### 2. Results

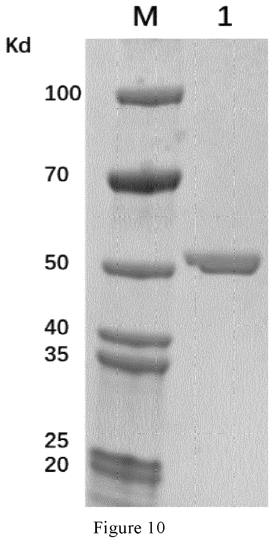

Figure 10 shows an SDS-PAGE gel electrophoresis image of purified Mph MP1 (variant of SEQ ID NO: 11).

### Example 2

This Example illustrates the capabilities of Pif1-like helicase to unwind hybridized dsDNA by using a fluorescence assay to detect enzyme activity.

### 1. Materials and methods

In Figure 1, as shown in a), the fluorescent substrate strand (100 nM final) has a 5' ssDNA overhang, and a 50 base section of hybridized dsDNA. The major upper strand has a black-hole quencher (BHQ-1) base (SEQ ID NO:20-BHQ-3') at the 3' end, and the hybridized complement has a carboxyfluorescein base (5'FAM-SEQ ID NO:21) at the 5' end. When the fluorescence from the fluorescein is quenched by the local BHQ-1, and the substrate is essentially non-fluorescent. 0.5 µM of a capture strand (SEQ ID NO:22) that is complementary to the shorter strand of the fluorescent substrate is also included. As shown in b), in the presence of ATP (5 mM) and MgCl₂ (5 mM), helicase (200 nM) added to the substrate binds to the 5' part of the fluorescent substrate, moves along the major strand, and unwinds the complementary strand. After that, excess of capture strand preferentially anneals to the complementary DNA to prevent re-annealing of initial substrate and loss of fluorescence. A certain amount of hybrid dsDNA that is not unwound by Pifl-like helicase is present in the system. As shown in c), after adding excessive capture strand A (SEQ ID NO:23) that is completely complementary to the major strand, part of the entangled dsDNA will have a strand unwinding effect due to the presence of excessive A, and finally all the dsDNA will be untwisted, and the fluorescence value will reach the highest.

### 2. Results

Figure 2 shows a graph of the time-dependent dsDNA unwinding ratio in a buffer containing 400mM NaCl (10 mM Hepes pH 8.0, 5 mM ATP, 5 mM MgCl₂, 100 nM fluorescent substrate DNA, 0.5 µM capture DNA).

### Example 3

This example shows an exemplary DNA binding capabilities of modified Pifl-like helicase of the present application by using gel measurements.

Specifically, DNA binding capabilities of Aph Acj61-D97C/A363C (SEQ ID NO: 2 with D97C/A363C mutation), Aph PX29-D96C/A371C (SEQ ID NO: 3 with D96C/A371C mutation), Sph CBH8-A94C/A361C/C136A (SEQ ID NO: 5 with A94C/A361C and C136A mutations) and Pph PspYZU05-D104C/A375C/C146A (SEQ ID NO: 8 with D104C/A375C and C146A mutations) and Mph MP1-E105C/A362C (SEQ ID NO: 11 with E105C/A362C mutant) were measured.

### 1. Materials and methods

The annealed DNA complex (hybridization of SEQ ID NO: 18 with SEQ ID NO: 19 modified with 5'FAM) were mixed with Aph Acj61, Aph PX29, Sph CBH8, Pph PspYZU05, Mph MP1, Aph Acj61-D97C/A363C, Aph PX29-D96C/A371C, Sph CBH8-A94C/A361C/C136A, Pph PspYZU05-D104C/A375C/A375C/MPC/A375C E105C/A362C, respectively, in a ratio of (1:1, volume/volume) in 10mM HEPE, pH 8.0, and 400mM potassium chloride to reach a final concentration of Pifl-like helicase of 600nM and a final concentration of DNA of 30nM. The Pifl-like helicase was allowed to bind to DNA for 1 hour at room temperature. To each sample TMAD was added to a final concentration of 5 µM, and incubated at room temperature for 1 hour. The sample was loaded on a 4-20% TBE gel and run the gel at 160V for 1.5 hours. The gel was placed under blue fluorescence to observe the DNA bands.

### 2. Results

Figure 3 shows the effect of modifications on the DNA-binding capabilities of Pifl-like helicases. Lanes 2 to 6 show that a large amount of DNA was not bound by the Pifl-like helicase during electrophoresis and no obvious binding bands are observed. Lanes 7-11 show the binding bands of different numbers of enzymes bound to DNA, and lanes 9 and 10 show that up to 5 Pifl-like helicases could bind to the single-stranded moiety of SEQ ID NO:18. This indicates that the modified Pifl-like helicase significantly enhanced its binding strength to DNA.

### Example 4

This example describes how a Mph MP1-E105C/A362C (SEQ ID NO: 11 with mutation E105C/A362C) controlled the movement of intact DNA strands through a single MspA nanopore (SEQ ID NO: 12).

### 1. Materials and methods

DNA construct A as shown in Figure 4 was prepared. SEQ ID NO: 13 was attached at its 5' end to twenty iSpC3 spacers and at its 3' end to four iSpC3 spacers which were attached to the 5' end of SEQ ID NO: 14 which was attached at its 3' end to SEQ ID NO: 17, and the SEQ ID NO: 15 region of this construct was hybridized to SEQ ID NO: 16 (having a 3' cholesterol tether).

The prepared DNA construct and Mph MP1-E105C/A362C were pre-incubated together for 30 minutes at 25°C in a buffer (10 mM HEPES, pH 8.0, 400 mM NaCl, 5% glycerol, 2 mM DTT).

Electrical measurements were acquired from MspA nanopores inserted in 1,2-diphytanoyl-glycero-3-phosphocholine lipid bilayers. Bilayers were formed across about 25 µm diameter apertures on a PTFE film via the Montal-Mueller technique, separating two 100 µL buffered solutions. All experiments were carried out in the stated buffered solution. Single-channel currents were measured on an amplifier equipped with digitizers. Ag/AgCl electrodes were connected to the buffered solutions so that the *cis* compartment is connected to the ground of the amplifier, and the *trans* compartment is connected to the active electrode of the amplifier.

After achieving a single pore in the bilayer, DNA polynucleotide and the Pifl-like helicase were added to 70 µL of buffer in the *cis* compartment of the electrophysiology chamber to initiate capture of the helicase-DNA complexes in the nanopore. Helicase ATPase activity was initiated as required by the addition of divalent metal (5 mM MgCl₂) and NTP (2.86µM ATP) to the *cis* compartment. Experiments were carried out at a constant potential of +180 mV.

### Results and Discussion

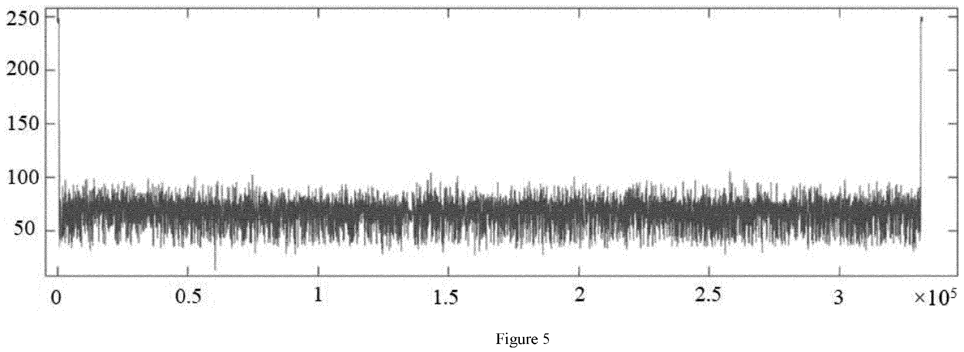

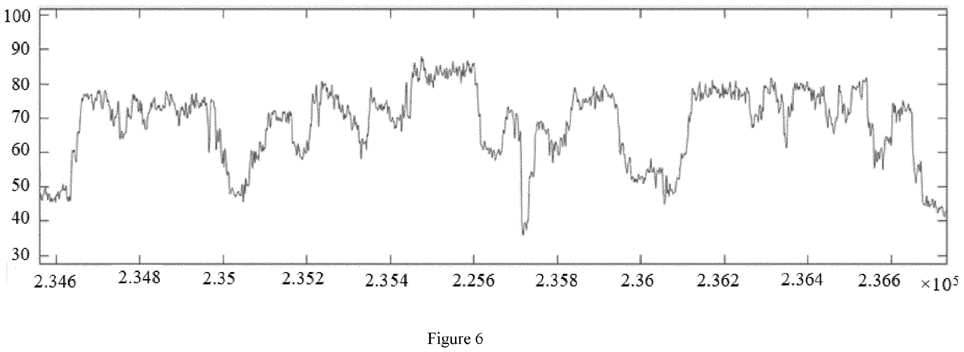

DNA movement controlled by the Pifl-like helicase was observed for the DNA construct. Observation of Pifl-like helicase-controlled DNA movement is shown in Figure 5. The Pifl-like helicase-controlled DNA movement was 50 seconds long and corresponded to approximately 10 kB of the DNA construct's translocation through the nanopore. Figure 6 shows an enlarged graph of a partial region of the Pifl-like helicase-controlled DNA movement.

### Example 5

This example describes how Sph CBH8-A94C/A361C/C136A (SEQ ID NO: 5 with A94C/A361C/C136A mutation), Eph Pei26-D99C/A366C/C141A (SEQ ID NO: 6 with D99C/A366C/C141A mutation) and Pph PspYZU05-D104C/A375C/C146A (SEQ ID NO: 8 with D104C/A375C and C146A mutations) controlled the movement of intact DNA strands through a single MspA nanopore.

### 1. Materials and methods

DNA construct B as shown in Figure 4 was prepared. SEQ ID NO: 13 was attached at its 5' end to twenty iSpC3 spacers and at its 3' end to four iSpC3 spacers which were attached to the 5' end of SEQ ID NO: 14 which was attached at its 3' end to SEQ ID NO: 24, and the SEQ ID NO: 15 region of this construct was hybridized to SEQ ID NO: 16 (having a 3' cholesterol tether). This DNA construct B was similar to the construct used in Example 4, except that the region labeled E corresponded to SEQ ID NO: 24.

DNA construct B in a buffer (in 50 mM NaCl, 10 mM Tris pH 7.5) and Sph CBH8-A94C/A361C/C136A, Eph Pei26-D99C/A366C/C141A or Pph PspYZU05-D104C/A375C/C146A in a buffer (50 mM KCl, 10 mM HEPES, pH 8.0) were pre-incubated together for 30 minutes at room temperature. TMAD was then added to the DNA/enzyme pre-mix and incubated for a further 30 minutes. Finally, a buffer (10 mM HEPES, 600 mM KCl, pH 8.0, 3 mM MgCl₂) and ATP were added to the pre-mix.

Electrical measurements were acquired at room temperature from single MspA nanopore inserted in block co-polymer in a buffer (10 mM HEPES, 400 mM KCl, pH 8.0). After achieving a single pore inserted in the block co-polymer, the pre-mix of the Pifl-like helicase (Sph CBH8-A94C/A361C/C136A, Eph Pei26-D99C/A366C/C141A or Pph PspYZU05-D104C/A375C/C146A (1nM final)), DNA (0.3nM final), fuel (ATP, 3mM final) was then added to the single nanopore experimental system. Each experiment was carried out for 2 hours at a holding potential of 180mV and helicase-controlled DNA movement was monitored.

### 2. Results

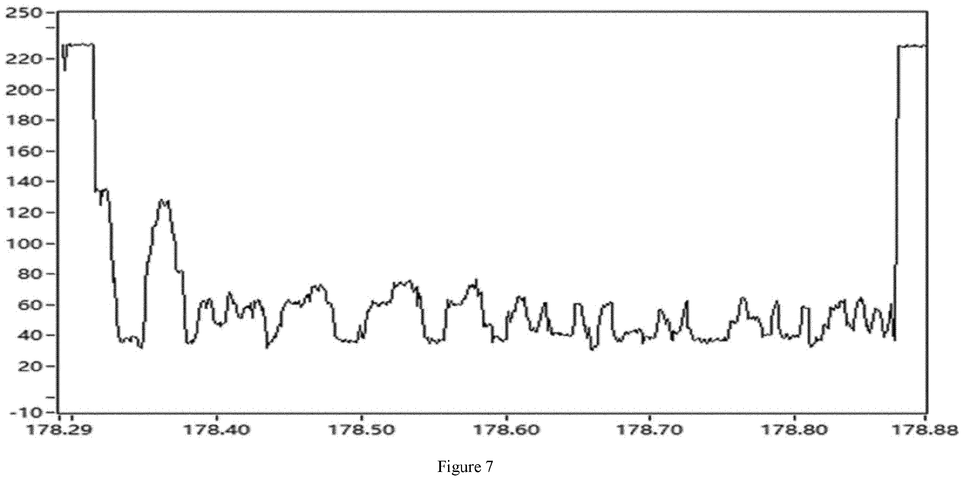

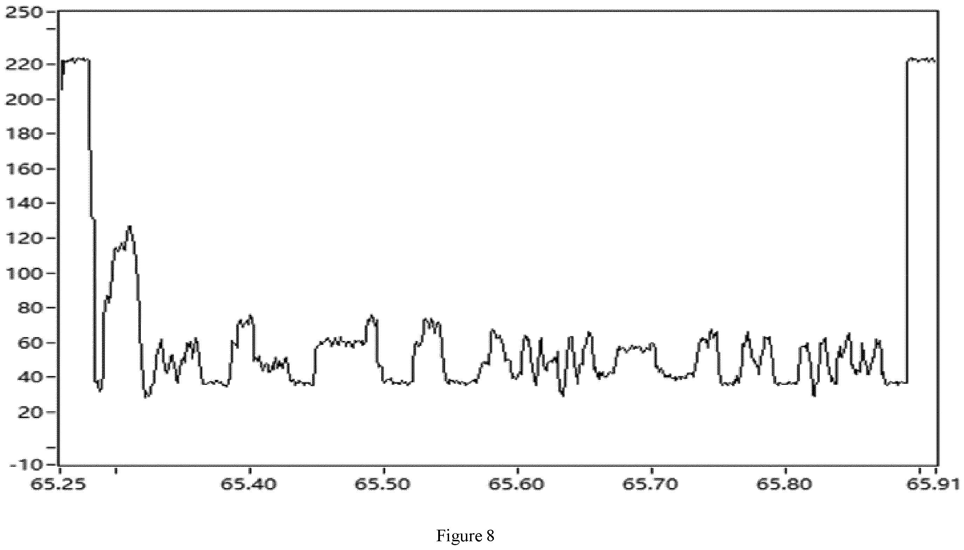

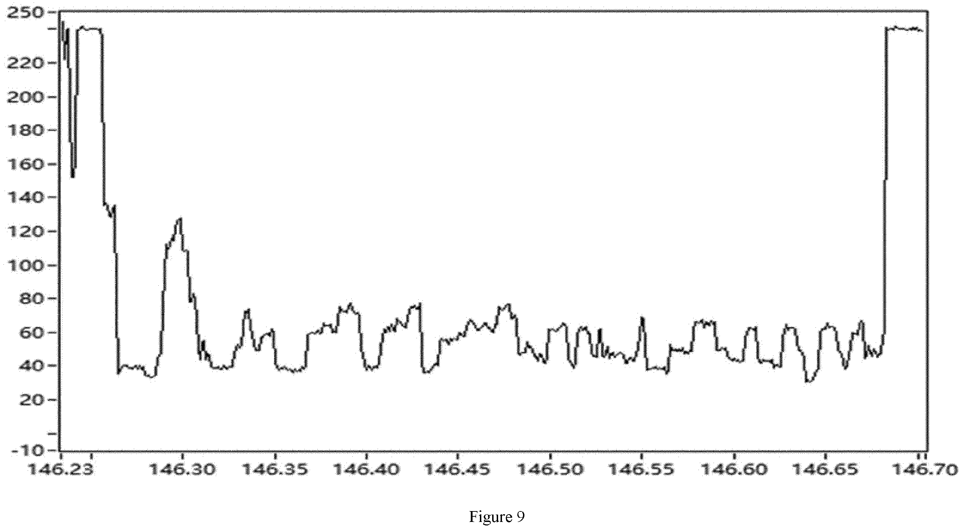

DNA movement controlled by Pifl-like helicase was observed for the DNA construct B. Observations of Sph CBH8-A94C/A361C/C136A, Eph Pei26-D99C/A366C/C141A or Pph PspYZU05-D104C/A375C/C146A-controlled DNA movements are shown in Figures 7-9, respectively.

The preferred embodiments of the present application are described in detail above. However, the present application is not limited to the specific details in the above embodiments. Within the scope of the technical concept of the present application, various simple variations could be made to the technical solution of the present application, and these simple variations belong to the protection scope of claims.

In addition, it should be noted that the various specific technical features described in the above specific embodiments can be combined in any suitable manner without contradiction. In order to avoid unnecessary repetition, the present application does not specify any possible combination mode separately.

## Claims

1. A Pifl-like helicase in which at least one cysteine residue and/or at least one non-natural amino acid have been introduced into a tower domain, a pin domain and/or a 1A domain of the Pifl-like helicase, wherein the Pifl-like helicase retains its ability to control the movement of a polynucleotide.

2. The Pifl-like helicase according to claim 1, wherein the helicase comprises:
(a) a variant of SEQ ID NO: 1 in which at least one cysteine residue and/or at least one non-natural amino acid have been introduced into residues E264-P278 and N296-A394 of the tower domain, and/or residues K89-E105 of the pin domain, and/or residues M1-L88 and M106-V181 of the 1A domain;
(b) a variant of SEQ ID NO: 2 in which at least one cysteine residue and/or at least one non-natural amino acid have been introduced into residues E265-P279 and N297-A392 of the tower domain, and/or residue K89-D105 of the pin domain, and/or residue M1-L88 and I106-M180 of the 1A domain;
(c) a variant of SEQ ID NO: 3 in which at least one cysteine residue and/or at least one non-natural amino acid have been introduced into residues T266-P280 and N298-S403 of the tower domain, and/or residues K89-A109 of the pin domain, and/or residues M1-L88 and K110-V182 of the 1A domain;
(d) a variant of SEQ ID NO: 4 in which at least one cysteine residue and/or at least one non-natural amino acid have been introduced into residues T266-P280 and N298-S404 of the tower domain, and/or residues K89-A109 of the pin domain, and/or residues M1-L88 and K110-V182 of the 1A domain;
(e) a variant of SEQ ID NO: 5 in which at least one cysteine residue and/or at least one non-natural amino acid have been introduced into residues E260-P274 and N292-A391 of the tower domain, and/or residues K86-E102 of the pin domain, and/or residues M1-L84 and M103-K177 of the 1A domain;
(f) a variant of SEQ ID NO: 6 in which at least one cysteine residue and/or at least one non-natural amino acid have been introduced into residues E266-P280 and N298-A396 of the tower domain, and/or residues K91-E107 of the pin domain, and/or residues M1-L90 and M108-M183 of the 1A domain;
(g) a variant of SEQ ID NO: 7 in which at least one cysteine residue and/or at least one non-natural amino acid have been introduced into residues T276-P290 and N308-P402 of the tower domain, and/or residues K100-D116 of the pin domain, and/or residues M1-L99 and D117-M191 of the 1A domain;
(h) a variant of SEQ ID NO: 8 in which at least one cysteine residue and/or at least one non-natural amino acid have been introduced into residues D274-P288 and N306-A404 of the tower domain, and/or residues K95-E112 of the pin domain, and/or residues M1-L95 and I113-K187 of the 1A domain;
(i) a variant of SEQ ID NO: 9 in which at least one cysteine residue and/or at least one non-natural amino acid have been introduced into residues E260-P274 and N292-A391 of the tower domain, and/or residues K86-E102 of the pin domain, and/or residues M1-L85 and M103-K177 of the 1A domain;
(j) a variant of SEQ ID NO: 10 in which at least one cysteine residue and/or at least one non-natural amino acid have been introduced into residues E265-P279 and H297-A393 of the tower domain, and/or residues K88-E104 of the pin domain, and/or residues M1-L87 and I105-K180 of the 1A domain; or
(k) a variant of SEQ ID NO: 11 in which at least one cysteine residue and/or at least one non-natural amino acid have been introduced into residues E264-P278 and N296-P389 of the tower domain, and/or residues K97-A113 of the pin domain, and/or residues M1-L96 and P114-K184 of the 1A domain.

3. The Pifl-like helicase according to claim 2, wherein the helicase comprises:
(a) a variant of SEQ ID NO: 11, which comprises (i) E105C and/or A362C; (ii) E104C and/or K360C; (iii) E104C and/or A362C; (iv) E104C and/or Q363C; (v) E104C and/or K366C; (vi) E105C and/or M356C; (vii) E105C and/or K360C; (viii) E104C and/or M356C; (ix) E105C and/or Q363C; (x) E105C and/or K366C; (xi) F108C and/or M356C; (xii) F108C and/or K360C; (xiii) F108C and/or A362C; (xiv) F108C and/or Q363C; (xv) F108C and/or K366C; (xvi) K134C and/or M356C; (xvii) K134C and/or K360C; (xviii) K134C and/or A362C; (xix) K134C and/or Q363C; (xx) K134C and/or K366C; (xxi) any of (i) to (xx) and G359C; (xxii) any of (i) to (xx) and Q111C; (xxiii) any of (i) to (xx) and I138C; (xxiv) any of (i) to (xx) and Q111C and I138C; (xxv) E105C and/or F377C; (xxvi) Y103L, E105Y, N352N, A362C and Y365N; (xxvii) E105Y and A362C; (xxviii) A362C; (xxix) Y103L, E105C, N352N, A362Y and Y365N; (xxx) Y103L, E105C and A362Y; (xxxi) E105C and/or A362C, and I280A; (xxxii) E105C and/or L358C; (xxxiii) E104C and/or G359C; (xxxiv) E104C and/or A362C ; (Xxxv) K106C and/or W378C; (xxxvi) T102C and/orN382C; (xxxvii) T102C and/or W378C; (xxxviii) E104C and/or Y355C; (xxxix) E104C and/or N382C; (xl) E104C and/or K381C; (xli) E104C and/or K379C; (xlii) E104C and/or D376C; (xliii) E104C and/or W378C; (xliv) E104C and/or W374C; (xlv) E105C and/or Y355C; (xlvi) E105C and/or N382C; (xlvii) E105C and/or K381C; (xlviii) E105C and/or K379C; (xlix) E105C and/or D376C; (1) E105C and/or W378C; (li) E105C and/or W374C; (lii) E105C and A362Y; (liii) E105C, G359C and A362C; or (liv)I2C, E105C and A362C; or,
(b) a variant of any one of SEQ ID NOs: 1 to 10, which comprises a cysteine residue at the positions which correspond to those in SEQ ID NO: 11 as defined in any of (i) to (liv).

4. The Pifl-like helicase according to any one of claims 1 to 3, wherein the amino acid sequence of the Pifl-like helicase is any of the amino acid sequences shown in SEQ ID NOs:1 to 11 or has at least 30%, at least 40%, at least 50%, 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology with any of the amino acid sequences shown in SEQ ID NOs: 1 to 11, and has the ability to control the movement of a polynucleotide.

5. The Pifl-like helicase according to any one of claims 1 to 4, wherein the introduced cysteines are connected to one another, the introduced non-natural amino acids are connected to one another, the introduced cysteine and the introduced non-natural amino acid are connected to one another, the introduced cysteine and the native amino acid are connected to one another, or the introduced non-natural amino acid and the native amino acid are connected to one another.

6. The Pifl-like helicase according to any one of claims 1 to 5, wherein the Pifl-like helicase further comprises substitution of at least one or more cysteine residues; preferably the cysteine residues are substituted with alanine, serine or valine.

7. The Pifl-like helicase according to any one of claims 1 to 6, wherein the Pifl-like helicase comprises:
(a) a variant of SEQ ID NO: 5, and the one or more substituted native cysteine residues are one or more of C109, C114, C136 or C414; or,
(b) a variant of any one of SEQ ID NOs: 1, 2, 3, 4, 6, 7, 8, 9, 10, and 11, and the one or more substituted native cysteine residues correspond to one or more of C109, C114, C136 or C414 in SEQ ID NO:5.

8. The Pifl-like helicase according to any one of claims 1 to 7, wherein the Pifl-like helicase further comprises substitution or modification of surface negatively-charged amino acids, polar or non-polar amino acids; more preferably, the substitution comprises substitution of negatively charged amino acids, uncharged amino acids, aromatic amino acids, polar or non-polar amino acids with positively charged amino acids, or uncharged amino acids.

9. The Pif1-like helicase of claim 8, wherein the Pif1-like helicase comprises:
a) a variant of SEQ ID NO: 11, and the one or more negatively charged amino acids are one or more of D5, E9, E24, E87, I65, S58, D209 or D216; or,
b) a variant of any one of SEQ ID NOs: 1 to 10, and the one or more negatively charged amino acids correspond to one or more of D5, E9, E24, E87, I65, S58, D209, or D216 in SEQ ID NO: 11.

10. The Pifl-like helicase according to claim 9, wherein the Pifl-like helicase further comprises:
a) a variant of SEQ ID NO: 1, and the one or more negatively charged amino acids further comprise S171; or,
b) a variant of SEQ ID NO: 9, and the one or more negatively charged amino acids further comprise S173.

11. The Pifl-like helicase according to any one of claims 1 to 10, wherein the non-natural amino acid is selected from 4-Azido-L-phenylalanine (Faz), 4-Acetyl-L-phenylalanine, 3-Acetyl-L-phenylalanine, 4-Acetoacetyl-L-phenylalanine, O-Allyl-L-tyrosine, 3-(Phenylselanyl)-L-alanine, O-2-Propyn-l-yl-L-tyrosine, 4-(Dihydroxyboryl)-L-phenylalanine, 4-[(Ethylsulfanyl)carbonyl]-L-phenylalanine, (2*S*)-2-amino-3-{4-[(propan-2-ylsulfanyl)carbonyl]phenyl}propanoic acid, (2*S*)-2-amino-3-{4-[(2-amino-3-sulfanylpropanoyl)amino]phenyl}propanoic acid, O-Methyl-L-tyrosine, 4-Amino-L-phenylalanine, 4-Cyano-L-phenylalanine, 3-Cyano-L-phenylalanine, 4-Fluoro-L-phenylalanine, 4-Iodo-L-phenylalanine, 4-Bromo-L-phenylalanine, O-(Trifluoromethyl)tyrosine, 4-Nitro-L-phenylalanine, 3-Hydroxy-L-tyrosine, 3-Amino-L-tyrosine, 3-Iodo-L-tyrosine, 4-Isopropyl-L-phenylalanine, 3-(2-Naphthyl)-L-alanine, 4-Phenyl-L-phenylalanine, (2*S*)-2-amino-3-(naphthalen-2-ylamino)propanoic acid, 6-(Methylsulfanyl)norleucine, 6-Oxo-L-lysine, D-tyrosine, (2R)-2-Hydroxy-3-(4-hydroxyphenyl)propanoic acid, (2R)-2-Ammoniooctanoate3-(2,2'-Bipyridin-5-yl)-D-alanine, 2-amino-3-(8-hydroxy-3-quinolyl)propanoic acid, 4-Benzoyl-L-phenylalanine, S-(2-Nitrobenzyl)cysteine, (2R)-2-amino-3-[(2-nitrobenzyl)sulfanyl]propanoic acid, (2*S*)-2-amino-3-[(2-nitrobenzyl)oxy]propanoic acid, O-(4,5-Dimethoxy-2-nitrobenzyl)-L-serine, (2*S*)-2-amino-6-({[(2-nitrobenzyl)oxy]carbonyl}amino)hexanoic acid, O-(2-Nitrobenzyl)-L-tyrosine, 2-Nitrophenylalanine, 4-[(E)-Phenyldiazenyl]-L-phenylalanine, 4-[3-(Trifluoromethyl)-3H-diaziren-3-yl]-D-phenylalanine, 2-amino-3-[[5-(dimethylamino)-l-naphthyl]sulfonylamino]propanoic acid, (2*S*)-2-amino-4-(7-hydroxy-2-oxo-2H-chromen-4-yl)butanoic acid, (2*S*)-3-[(6-acetylnaphthalen-2-yl)amino]-2-aminopropanoic acid, 4-(Carboxymethyl)phenylalanine, 3-Nitro-L-tyrosine, O-Sulfo-L-tyrosine, (2R)-6-Acetamido-2-ammoniohexanoate, 1-Methylhistidine, 2-Aminononanoic acid, 2-Aminodecanoic acid, L-Homocysteine, 5-Sulfanylnorvaline, 6-Sulfanyl-L-norleucine, 5-(Methyl sulfanyl)-L-norvaline, N⁶-{[(2R,3R)-3-Methyl-3,4-dihydro-2H-pyrrol-2-yl]carbonyl}-L-lysine, N⁶-[(Benzyloxy)carbonyl]lysine, (2*S*)-2-amino-6-[(cyclopentylcarbonyl)amino]hexanoic acid, N⁶-[(Cyclopentyloxy)carbonyl]-L-lysine, (2*S*)-2-amino-6-{[(2*R*)-tetrahydrofuran-2-ylcarbonyl]amino}hexanoic acid, (2*S*)-2-amino-8-[(2*R*,3*S*)-3-ethynyltetrahydrofuran-2-yl]-8-oxooctanoic acid, N⁶-(tert-Butoxycarbonyl)-L-lysine, (2S)-2-Hydroxy-6-({[(2-methyl-2-propanyl)oxy]carbonyl}amino)hexanoic acid, N⁶-[(Allyloxy)carbonyl]lysine, (2*S*)-2-amino-6-({[(2-azidobenzyl)oxy]carbonyl}amino)hexanoic acid, N⁶-L-Prolyl-L-lysine, (2*S*)-2-amino-6-{[(prop-2-yn-l-yloxy)carbonyl]amino}hexanoic acid and N⁶-[(2-Azidoethoxy)carbonyl]-L-lysine.

12. The Pif1-like helicase according to any one of claims 1 to 11, wherein the Pif1-like helicase further comprises:
(a) substitution of at least one amino acid that interacts with one or more nucleotides in single-stranded DNA or double-stranded DNA; and/or,
(b) substitution of at least one amino acid that interacts with a transmembrane pore, wherein the Pifl-like helicase has the ability to control the movement of a polynucleotide.

13. The Pifl-like helicase according to claim 12, wherein in (a), at least one amino acid that interacts with a sugar ring and/or base of one or more nucleotides in single-stranded DNA or double-stranded DNA is substituted with an amino acid containing a larger side chain.

14. The Pifl-like helicase according to claim 13, wherein the Pifl-like helicase comprises:
(a) a variant of SEQ ID NO: 11, wherein the at least one amino acid that interacts with a sugar ring and/or base of one or more nucleotides in single-stranded DNA or double-stranded DNA is at least one of P73, H93, N99, F109, 1280, A161, F130, D132, D162, D163, E277, K415, Q291, H396, Y244 or P100; or,
(b) a variant of any one of SEQ ID NOs: 1 to 10, wherein the at least one amino acid that interacts with a sugar ring and/or base of one or more nucleotides in single-stranded DNA or double-stranded DNA corresponds to at least one of P73, H93, N99, F109, 1280, A161, F130, D132, D162, D163, E277, K415, Q291, H396, Y244 or P100 in SEQ ID NO: 11.

15. The Pifl-like helicase according to claim 13 or 14, wherein the larger side chain comprises an increased number of carbon atoms, has an increased length, an increased molecular volume, and/or has an increased van der Waals volume.

16. The Pifl-like helicase according to any one of claims 13 to 15, wherein the larger side chain increases (i) electrostatic interaction, (ii) hydrogen bonding and/or (iii) cation-pi interaction between the at least one amino acid and one or more nucleotides in the single-stranded DNA or double-stranded DNA.

17. The Pifl-like helicase according to any one of claims 13 to 16, wherein the amino acid containing the larger side chain is not alanine (A), cysteine (C), glycine (G) , selenocysteine (U), methionine (M), aspartic acid (D) or glutamic acid (E).

18. The Pifl-like helicase according to any one of claims 12 to 17, wherein:
A) histidine (H) is substituted with (i) arginine (R) or lysine (K); (ii) glutamine (Q) or asparagine (N); or (iii) phenylalanine (F), tyrosine (Y) or tryptophan (W);
B) asparagine (N) is substituted with (i) arginine (R) or lysine (K); (ii) glutamine (Q) or histidine (H); or (iii) phenylalanine (F), tyrosine (Y) or tryptophan (W);
C) proline (P) is substituted with (i) arginine (R) or lysine (K); (ii) glutamine (Q), asparagine (N), threonine (T) or histidine (H); (iii) tyrosine (Y), phenylalanine (F) or tryptophan (W); or (iv) leucine (L), valine (V) or isoleucine (I);
D) phenylalanine (F) is substituted with (i) arginine (R) or lysine (K); (ii) histidine (H); or (iii) tyrosine (Y) or tryptophan Acid (W);
E) aspartic acid (D) is substituted with (i) arginine (R) or lysine (K); (ii) glutamine (Q), asparagine (N) or histidine (H); or (iii) phenylalanine (F), tyrosine (Y) or tryptophan (W);
F) valine (V) is substituted with (i) arginine (R) or lysine (K); (ii) glutamine (Q), asparagine (N) or histidine (H); (iii) phenylalanine (F), tyrosine (Y) or tryptophan (W); or (iv) isoleucine (I) or leucine (L);
G) serine (S) is substituted with (i) arginine (R) or lysine (K); (ii) glutamine (Q), asparagine (N) or histidine (H); (iii) phenylalanine (F), tyrosine (Y) or tryptophan (W); or (iv) isoleucine (I) or leucine (L); and/or,
H) tyrosine (Y) is substituted with (i) arginine (R) or lysine (K); or (ii) tryptophan (W).

19. The Pifl-like helicase according to any one of claims 12 to 18, wherein the Pifl-like helicase is a variant of SEQ ID NO: 11 comprising:
P73L; P73V; P73I; P73E; P73T; P73F; H93N; H93Q; H93W; N99R; N99H; N99W; N99Y; P100L; P100V; P100I; P100E; P100T; P100F; F130W; F130Y; F130H; D132H; D132Y; D132K; A161I; A161L; A161N; A161W; A161H; D162H; D162Y; D162K; D163W; D163F; D163Y; D163H; D163I; D163L; D163V; Y244W; Y244H; E277G; I280H; I280K; I280W; Q291K; Q291R; Q291W; Q291F; H396N; H396Q; H396W; K415W; K415R; K415H; K415Y; F109W/P73L; F109W/P73V; F109W/P73I; F109W/P73E; F109W/P73T; F109W/P73F; F109W/H93N; F109W/H93Q; F109W/H93W; F109W/N99R; F109W/N99H; F109W/N99W; F109W/N99Y; F109W/P100L; F109W/P100V; F109W/P1001; F109W/P100E; F109W/P100T; F109W/P100F; F109W/F130W; F109W/F130Y; F109W/F130H; F109W/D132H; F109W/D132Y; F109W/D132K; F109W/A161I; F109W/A161L; F109W/A161N; F109W/A161W; F109W/A161H; F109W/D162H; F109W/D162Y; F109W/D162K; F109W/D163W; F109W/D163F; F109W/D163Y; F109W/D163H; F109W/D163I; F109W/D163L; F109W/D163V; F109W/Y244W; F109W/Y244H; F109W/E277G; F109W/I280H; F109W/I280K; F109W/I280W; F109W/Q291K; F109W/Q291R; F109W/Q291W; F109W/Q291F; F109W/H396N; F109W/H396Q; F109W/H396W; F109W/K415W; F109W/K415R; F109W/K415H; or F109W/K415Y.

20. The Pifl-like helicase according to any one of claims 12 to 19, wherein, in (a), at least one amino acid that interacts with one or more phosphate groups of one or more nucleotides in single-stranded DNA or double-stranded DNA is substituted.

21. The Pifl-like helicase of claim 20, wherein the Pifl-like helicase comprises:
(a) a variant of SEQ ID NO: 11, in which the at least one amino acid that interacts with one or more phosphate groups of one or more nucleotides in ssDNA or dsDNA is at least one of H75, T91, S94, K97, N246, N247, N284, K288, N297, T394 or K397; or,
(b) a variant of any one of SEQ ID NOs: 1 to 10, in which the at least one amino acid that interacts with one or more phosphate groups of one or more nucleotides in ssDNA or dsDNA is at least one amino acid corresponding to H75, T91, S94, K97, N246, N247, N284, K288, N297, T394 or K397 in SEQ ID NO: 11.

22. The Pifl-like helicase according to claim 21, wherein:
a) histidine (H) is substituted with (i) arginine (R) or lysine (K); (ii) asparagine (N), serine (S), glutamine (Q) or threonine (T); or (iii) phenylalanine (F), tryptophan (W) or tyrosine (Y);
b) threonine (T) is substituted with (i) arginine (R), histidine (H) or lysine (K); (ii) asparagine (N), serine (S), glutamine (Q) or histidine (H); or (iii) phenylalanine (F), tryptophan (W), tyrosine (Y) or histidine (H);
c) serine is substituted with (i) arginine (R), histidine (H) or lysine (K); (ii) asparagine (N), glutamine (Q), threonine (T) or histidine (H); or (iii) phenylalanine (F), tryptophan (W), tyrosine (Y) or histidine (H);
d) asparagine (N) is substituted with (i) arginine (R), histidine (H) or lysine (K); (ii) serine (S), glutamine (Q), threonine (T) or histidine (H); or (iii) phenylalanine (F), tryptophan (W), tyrosine (Y) or histidine (H); and/or,
e) lysine (K) is substituted with (i) arginine (R) or histidine (H); (ii) asparagine (N), serine (S), glutamine (Q), threonine (T) or histidine (H); or (iii) phenylalanine (F), tryptophan (W), tyrosine (Y) or histidine (H).

23. The Pifl-like helicase according to any one of claims 20 to 22, wherein the Pifl-like helicase is a variant of SEQ ID NO: 11 comprising one or more of (a) to (k),
(a) H75N, H75Q, H75K or H75F; (b) T91K, T91Q or T91N; (c) S94H, S94N, S94K, S94T, S94R or S94Q; (d) K97Q, K97H or K97Y; (e) N246H or N246Q ; (f) N247Q or N247H; (g) N284H or N284Q; (h) K288Q or K288H; (i) N297Q, N297K or N297H; (j) T394K, T394H or T394N; or (k) K397R, K397H or K397Y.

24. The Pifl-like helicase according to any one of claims 12 to 23, wherein the helicase comprises substitution of at least one amino acid that interacts with the transmembrane pore:
(a) a variant of SEQ ID NO: 11, in which the Pifl-like helicase comprises one or more substitutions on (a) E196 (b) W202 (c) N199 or (d) G201; or,
(b) a variant of any one of SEQ ID NOs: 1 to 10, in which the Pifl-like helicase comprises at least one substitutions on amino acid corresponding to (a) E196 (b) W202 (c) N199 or (d) G201.

25. The Pifl-like helicase according to any one of claims 1-24, wherein the Pifl-like helicase is a variant of SEQ ID NO: 11 comprising substitutions at the following positions:
- F109/E196/H75, such as, F109W/E196L/H75N, F109W/E196L/H75Q, F109W/E196L/H75K or F109W/E196L/H75F;
- F109/E196/T91, such as, F109W/E196L/T91K, F109W/E196L/T91Q or F109W/E196L/T91N;
- F109/S94/E196, such as, F109W/S94H/E196L, F109W/S94T/E196L, F109W/S94R/E196L, F109W/S94Q/E196L, F109W/S94N/E196L or F109W/S94K/E196L;
- F109/N99/E196, such as, F109W/N99R/E196L, F109W/N99H/E196L, F109W/N99W/E196L or F109W/N99Y/E196L;
- F109/S94/E196/1280, such as, F109W/S94H/E196L/I280K;
- F109/P100/E196, such as, F109W/P100L/E196L, F109W/P100V/E196L, F109W/P100I/E196L or
F109W/P100T/E196L;
- F109/D132/E196, such as, F109W/D132H/E196L, F109W/D132Y/E196L or F109W/D132K/E196L;
- F109/A161/E196, such as, F109W/A161I/E196L, F109W/A161L/E196L, F109W/A161N/E196L, F109W/A161W/E196L or F109W/A161H/E196L;
- F109/D163/E196, such as, F109W/D163W/E196L, F109W/D163F/E196L, F109W/D163Y/E196L, F109W/D163H/E196L, F109W/D163I/E196L, F109W/D163L/E196L or F109W/D163V/D163L/E196L;
- F109/Y244/E196, such as, F109W/Y244W/E196L, F109W/Y244Y/E196L or F109W/Y244H/E196L;
- F109/N246/E196, for example, F109W/N246H/E196L or F109W/N246Q/E196L;
- F109/E196/I280, such as, F109W/E196L/I280K, F109W/E196L/I280H, F109W/E196L/I280W or F109W/E196L/I280R;
- F109/E196/Q291, such as, F109W/E196L/Q291K, F109W/E196L/Q291R, F109W/E196L/Q291W or F109W/E196L/Q29IF;
- F109/N297/E196, such as, F109W/N297Q/E196L, F109W/N297K/E196L or F109W/N297H/E196L;
- F109/T394/E196, such as, F109W/T394K/E196L, F109W/T394H/E196L or F109W/T394N/E196L;
- F109/H396/E196, such as, F109W/H396Y/E196L, F109W/H396F/E196L, F109W/H396Q/E196L or F109W/H396K/E196L;
- F109/K397/E196, such as, F109W/K397R/E196L, F109W/K397H/E196L or F109W/K397Y/E196L; or,
- F109/Y416/E196, such as, F109W/Y416W/E196L or F109W/Y416R/E196L.

26. A construct comprising at least one Pifl-like helicase according to any one of claims 1 to 25.

27. The construct according to claim 26, wherein the construct further comprises a polynucleotide binding moiety.

28. A nucleic acid encoding the Pifl-like helicase according to any one of claims 1 to 25 or the construct according to any one of claims 26 to 27.

29. An expression vector comprising the nucleic acid according to claim 28.

30. A host cell comprising the nucleic acid according to claim 28 or the expression vector according to claim 29.

31. A method of controlling the movement of a polynucleotide, comprising contacting the Pifl-like helicase according to any one of claims 1 to 25 or the construct according to any one of claims 26 to 27 with the polynucleotide.

32. A method of characterising a target polynucleotide, comprising:
I) contacting the Pifl-like helicase according to any one of claims 1 to 25 or the construct according to any one of claims 26 to 27 with the target polynucleotide and a pore, such that the Pifl-like helicase or the construct controls the movement of the target polynucleotide through the pore; and
II) taking one or more characteristics of the target polynucleotide when the nucleotide of the target polynucleotide interacts with the pore, and thereby characterising the target polynucleotide.

33. The method according to claim 32, wherein the method further comprises the step of applying a potential difference across the pore contacting the helicase or the construct and the target polynucleotide; preferably, the pore is selected from a biological pore, a solid state pore, or a biological and solid state hybrid pore.

34. The method according to claim 32 or 33, wherein the target polynucleotide is single-stranded, double-stranded, or at least partially double-stranded.

35. The method according to any one of claims 32 to 34, wherein the one or more characteristics are selected from the source, length, identity, sequence, secondary structure of the target polynucleotide, or whether or not the target polynucleotide is modified; preferably, the one or more characteristics are measured by electrical measurement and/or optical measurement.

36. A product for characterising a target polynucleotide comprising the Pifl-like helicase according to any one of claims 1 to 25, the construct according to any one of claims 26 to 27, the nucleic acid according to claim 28, the expression vector according to claim 29 or the host cell according to claim 30, and a pore.

37. The product of claim 36, wherein the product is selected from a kit, a device or a sensor.

38. Use of the Pifl-like helicase according to any one of claims 1 to 25, the construct according to any one of claims 26 to 27, the nucleic acid according to claim 28, the expression vector according to claim 29, the host cell according to claim 30 or the product according to any one of claims 36 to 37 in characterising a target polynucleotide or controlling the movement of a target polynucleotide through a pore.
